# EUROPEAN PATENT SPECIFICATION

(11) **EP 2 959 840 B1**
(45) Date of publication and mention of the grant of the patent: **21.02.2018**
(21) Application number: 15173804.4
(22) Date of filing: 25.06.2015
(51) Int. Cl.: A61B 17/068, A61B 17/072

(54) **HAND HELD SURGICAL HANDLE ASSEMBLY, SURGICAL ADAPTERS FOR USE BETWEEN SURGICAL HANDLE ASSEMBLY AND SURGICAL END EFFECTORS**
TRAGBARE CHIRURGISCHE GRIFFANORDNUNG, CHIRURGISCHE ADAPTER ZUR VERWENDUNG ZWISCHEN DER CHIRURGISCHEN GRIFFANORDNUNG UND CHIRURGISCHEN ENDEFFEKTOREN
ENSEMBLE A POIGNEE CHIRURGICALE PORTATIVE, ADAPTATEURS CHIRURGICAUX POUR UTILISATION ENTRE UN ENSEMBLE A POIGNEE CHIRURGICALE ET DES EFFECTEURS D'EXTREMITE CHIRURGICALE

(30) Priority: 26.06.2014 US 201462017599 P; 09.06.2015 US 201514734159
(43) Date of publication of application: 30.12.2015
(73) Proprietor: Covidien LP, Mansfield, MA 02048 (US)
(72) Inventor: ZERGIEBEL, Earl M., Guilford, CT Connecticut 06430 (US); CHOWANIEC, David, Rocky Hill, CT Connecticut 06067 (US); WILLIAMS, Ryan, New Hartford, CT Connecticut 06057 (US); FOWLER, David, Cheshire, CT Connecticut 06410 (US)
(74) Representative: Soames, Candida Jane

(56) References cited:
- EP-A1- 2 329 773
- EP-A2- 2 668 910

## Description

### BACKGROUND

### 1. Technical Field

The present disclosure relates to surgical devices and/or systems, surgical adapters and their methods of use. More specifically, the present disclosure relates to hand held powered surgical devices, surgical adapters and/or adapter assemblies for use between and for interconnecting the powered, rotating and/or articulating surgical device or handle assembly and an end effector for clamping, cutting and/or stapling tissue.

### 2. Background of Related Art

One type of surgical device is a linear clamping, cutting and stapling device. Such a device may be employed in a surgical procedure to resect a cancerous or anomalous tissue from a gastro-intestinal tract. Conventional linear clamping, cutting and stapling instruments include a pistol grip-styled structure having an elongated shaft and distal portion. The distal portion includes a pair of scissors-styled gripping elements, which clamp the open ends of the colon closed. In this device, one of the two scissors-styled gripping elements, such as the anvil portion, moves or pivots relative to the overall structure, whereas the other gripping element remains fixed relative to the overall structure. The actuation of this scissoring device (the pivoting of the anvil portion) is controlled by a grip trigger maintained in the handle.

In addition to the scissoring device, the distal portion also includes a stapling mechanism. The fixed gripping element of the scissoring mechanism includes a staple cartridge receiving region and a mechanism for driving the staples up through the clamped end of the tissue against the anvil portion, thereby sealing the previously opened end. The scissoring elements may be integrally formed with the shaft or may be detachable such that various scissoring and stapling elements may be interchangeable.

A number of surgical device manufacturers have developed product lines with proprietary drive systems for operating and/or manipulating the surgical device. In many instances the surgical devices include a handle assembly, which is reusable, and a disposable end effector or the like that is selectively connected to the handle assembly prior to use and then disconnected from the end effector following use in order to be disposed of or in some instances sterilized for re-use.

Many of the existing end effectors for use with many of the existing surgical devices and/or handle assemblies are driven by a linear force. For examples, end effectors for performing endo-gastrointestinal anastomosis procedures, end-to-end anastomosis procedures and transverse anastomosis procedures, each typically require a linear driving force in order to be operated. As such, these end effectors are not compatible with surgical devices and/or handle assemblies that use a rotary motion to deliver power or the like. A device showing the features defined in the preamble of claim 1 is disclosed in document EP-A-2 668 910.

Additionally, forces transmitted to the surgical device and/or handle assemblies during firing of the stapling mechanism of the end effector may cause unwanted over-articulation.

In order to make the linear driven end effectors compatible with surgical devices and/or handle assemblies that use a rotary motion to deliver power and reduce the amount of unwanted articulation during firing of staples, a need exists for adapters and/or adapter assemblies to interface between and interconnect the linear driven end effectors with the rotary driven surgical devices and/or handle assemblies sufficiently rigid to minimize over-articulation during firing.

### SUMMARY

The present disclosure relates to hand held powered surgical devices, surgical adapters and/or adapter assemblies for use between and for interconnecting the powered, rotating and/or articulating surgical device or handle assembly and an end effector for clamping, cutting and/or stapling tissue.

According to an aspect of the present disclosure, an adapter for selectively interconnecting a surgical end effector is provided. The adapter is configured to perform a function and includes a handle assembly that is configured to actuate the end effector. The end effector includes at least one axially translatable drive member, and the handle assembly includes at least one rotatable drive shaft. The adapter housing is configured and adapted for connection with the handle assembly and to be in operative communication with each rotatable drive shaft of the handle assembly. The adapter housing further includes an inner wall surface defining a cavity, wherein a flange projects radially inward from the inner wall surface on the distal end of the adapter housing. The adapter housing also includes an outer tube which tapers distally from a larger diameter to a smaller diameter. The outer tube is rotatably and translatably supported by the flange of the adapter housing and the distal end of the outer tube is configured and adapted for connection with the end effector and for operative communication with each axially translatable drive member of the end effector.

The adapter further includes an inner housing having an inner wall surface defining a cavity and an outer surface, wherein an annular groove is defined in the proximal end of the inner wall surface. The diameter of the outer surface of the inner housing is less than the diameter of the inner wall surface of the adapter housing such that the inner housing is rotatably supported in the adapter housing. The adapter housing further includes at least one drive converter assembly for interconnecting a respective one rotatable drive shaft of the handle assembly and one axially translatable drive member of the end effector. The at least one drive converter assembly includes a first end that is connectable to a rotatable drive shaft of the handle assembly and a second end that is connectable to the axially translatable drive member of the end effector such that wherein the at least one drive converter assembly converts and transmits a rotation of the rotatable drive shaft of the handle assembly to an axial translation of the axially translatable drive member of the end effector. Additionally, the at least one drive converter assembly is configured to be rotatably and translatably supported in the annular groove in the inner housing.

The inner housing of the adapter may be in mechanical cooperation with the outer tube.

The adapter may include a plurality of arms extending distally along the longitudinal axis from the inner housing that are in mechanical cooperation with the outer tube.

The plurality of arms of the adapter may comprise a pair of parallel arms.

The drive converter assembly of the at least one drive converter assembly of the adapter may also include a first drive converter assembly including a first distal drive shaft rotatably supported in the adapter housing, wherein a proximal end of the first distal drive shaft is connectable to the rotatable drive shaft of the handle assembly. The first drive converter assembly further includes a drive coupling nut threadably connected to a threaded distal portion of the first distal drive shaft, wherein the drive coupling nut is keyed against rotation within the adapter housing. The first drive converter assembly also includes a drive tube having a proximal end connected to the drive coupling nut and a distal end configured for selective engagement with the at least one axially translatable drive member of the end effector. Rotation of the rotatable drive shaft of the handle assembly results in rotation of the distal drive shaft, and rotation of the distal drive shaft results in axial translation of the drive coupling nut, the drive tube and the at least one axially translatable drive member of the end effector.

The first drive converter assembly of the adapter may include a spur gear keyed to the proximal end of the distal drive shaft; a proximal rotatable drive shaft having a spur gear supported on a distal end thereof and a proximal end connectable to the rotatable drive shaft of the handle assembly; and a compound gear interengaging the spur gear keyed to the proximal end of the distal drive shaft and the spur gear supported on the distal end of the proximal rotatable drive shaft.

In use, the translation of the at least one axially translatable drive member of the end effector results in a closing of the end effector and a firing of the end effector.

The at least one drive converter assembly of the adapter may also include a second drive converter assembly including a second proximal drive shaft rotatably supported in the adapter housing, wherein a proximal end of the second proximal drive shaft is connectable to a second rotatable drive shaft of the handle assembly; a coupling cuff rotatably and translatably supported in the adapter housing, the coupling cuff defining an inner annular race; a coupling slider rotatably disposed within the annular race of the coupling cuff, the coupling slider being threadably connected to a threaded distal portion of the second proximal drive shaft; and a drive bar having a proximal end connected to the coupling cuff and a distal end configured for selective engagement with another axially translatable drive member of the end effector. Rotation of the second rotatable drive shaft of the handle assembly results in rotation of the second proximal drive shaft, and rotation of the second proximal drive shaft results in axial translation of the coupling slider, the coupling cuff, the drive bar and the another axially translatable drive member of the end effector.

The first distal drive shaft may extend through the coupling cuff such that the coupling cuff is rotatable about the first distal drive shaft.

In use, translation of the another axially translatable drive member of the end effector results in an articulation of the end effector relative to the adapter.

The adapter may further include a drive transmitting assembly including a third proximal rotatable drive shaft rotatably supported in the adapter housing and having a spur gear supported on a distal end thereof and a proximal end connectable to a third rotatable drive shaft of the handle assembly; a ring gear rotatably supported in the adapter housing, the ring gear defining an internal array of gear teeth which are engaged with the spur gear of the third proximal rotatable drive shaft, wherein the ring gear is keyed to the inner housing; and at least one rotation transmitting bar having an proximal end connected to the inner housing and a distal end connected to a distal coupling assembly, wherein the distal coupling assembly is configured to selectively connect with the end effector. Rotation of the third rotatable drive shaft of the handle assembly results in rotation of the third proximal drive shaft, and rotation of the third proximal drive shaft results in rotation of the ring gear, the inner housing, the at least one rotation transmitting bar and the distal coupling assembly to rotate the end effector relative to the adapter and about a longitudinal axis defined by the adapter.

The outer tube of the adapter may be configured for endoscopic insertion into a target surgical site. The adapter housing may be inhibited from insertion into the target surgical site.

At least one of the first drive converter assembly, the second drive converter assembly or the drive transmitting assembly may be disposed in the adapter housing.

In embodiments, the end effector and the outer tube of the adapter define an endoscopic portion that is configured for endoscopic insertion into a target surgical site.

In embodiments, the end effector and the outer tube define an endoscopic portion that is configured for endoscopic insertion into a target surgical site. Each of the first drive converter assembly, and the second drive converter assembly, or the drive transmitting assembly may be disposed outside of the endoscopic portion.

According to an aspect of the present disclosure, an adapter for selectively interconnecting a surgical end effector that is configured to perform a function and a handle assembly that is configured to actuate the end effector is provided. The end effector includes at least one axially translatable drive member, and the handle assembly includes at least one rotatable drive shaft. The adapter includes an adapter housing configured and adapted for connection with the handle assembly and to be in operative communication with each rotatable drive shaft of the handle assembly, the adapter housing having an inner wall surface defining a cavity; and an outer tube having a proximal end and a distal end, wherein the proximal end is supported by the adapter housing, wherein the distal coupling assembly is configured and adapted for connection with the end effector and for operative communication with each axially translatable drive member of the end effector.

The adapter further includes an inner housing having proximal and distal ends defining a longitudinal axis, the inner housing having an inner wall surface defining a cavity and an outer surface, wherein a diameter of the outer surface of the inner housing is less than a diameter of the inner wall surface of the adapter housing, wherein the inner housing is rotatably supported in the adapter housing; and at least one drive converter assembly for interconnecting a respective one rotatable drive shaft of the handle assembly and one axially translatable drive member of the end effector.

The at least one drive converter assembly includes a first end that is connectable to a rotatable drive shaft of the handle assembly and a second end that is connectable to the axially translatable drive member of the end effector, wherein the at least one drive converter assembly converts and transmits a rotation of the rotatable drive shaft of the handle assembly to an axial translation of the axially translatable drive member of the end effector, wherein the at least one drive converter assembly is configured to be rotatably supported by the inner housing; an intermediate housing having proximal and distal ends defining a longitudinal axis the intermediate housing being fixably supported by the adapter housing, wherein the intermediate housing has an outer surface, the outer surface having a circumferential groove, wherein the diameter of the outer surface is less than the diameter of the inner surface of the adapter housing; and at least one coupling bar having a proximal and distal end, wherein the proximal end of the at least one coupling bar is rotatably and translatably supported by the groove in the intermediate housing and the distal end connected to the distal coupling assembly, wherein the proximal end of the at least one coupling bar is also in mechanical cooperation with the inner housing.

The at least one coupling bar of the adapter may include two parallel coupling bars.

The at least one coupling bar may include a boss on a proximal end thereof to be engaged by the groove in the intermediate housing.

The at least one drive converter assembly of the adapter may include a first drive converter assembly including a first distal drive shaft rotatably supported in the adapter housing, wherein a proximal end of the first distal drive shaft is connectable to the rotatable drive shaft of the handle assembly; a drive coupling nut threadably connected to a threaded distal portion of the first distal drive shaft, wherein the drive coupling nut is keyed against rotation within the adapter housing; and a drive tube having a proximal end connected to the drive coupling nut and a distal end configured for selective engagement with the at least one axially translatable drive member of the end effector. Rotation of the rotatable drive shaft of the handle assembly results in rotation of the distal drive shaft, and rotation of the distal drive shaft results in axial translation of the drive coupling nut, the drive tube and the at least one axially translatable drive member of the end effector.

The first drive converter assembly of the adapter may include a spur gear keyed to the proximal end of the distal drive shaft; a proximal rotatable drive shaft having a spur gear supported on a distal end thereof and a proximal end connectable to the rotatable drive shaft of the handle assembly; and a compound gear interengaging the spur gear keyed to the proximal end of the distal drive shaft and the spur gear supported on the distal end of the proximal rotatable drive shaft.

In use, translation of the at least one axially translatable drive member of the end effector results in a closing of the end effector and a firing of the end effector.

The at least one drive converter assembly of the adapter may include a second drive converter assembly including a second proximal drive shaft rotatably supported in the adapter housing, wherein a proximal end of the second proximal drive shaft is connectable to a second rotatable drive shaft of the handle assembly; a coupling cuff rotatably and translatably supported in the adapter housing, the coupling cuff defining an inner annular race; a coupling slider rotatably disposed within the annular race of the coupling cuff, the coupling slider being threadably connected to a threaded distal portion of the second proximal drive shaft; and a drive bar having a proximal end connected to the coupling cuff and a distal end configured for selective engagement with another radially translatably drive member of the end effector. Rotation of the second rotatably drive shaft of the handle assembly results in rotation of the second proximal drive shaft, and rotation of the second proximal drive shaft results in axial translation of the coupling slider, the coupling cuff, the drive bar and the another axially translatable drive member of the end effector.

The adapter may further includes a connector sleeve interconnecting the second rotatable drive shaft of the device with the second proximal drive shaft of the adapter.

In use, translation of the another axially translatable drive member of the end effector results in an articulation of the end effector relative to the adapter.

The adapter may further includes a drive transmitting assembly including a third proximal rotatable drive shaft rotatably supported in the adapter housing and having a spur gear supported on a distal end thereof and a proximal end connectable to a third rotatable drive shaft of the handle assembly; a ring gear rotatably supported in the adapter housing, the ring gear defining an internal array of gear teeth which are engaged with the spur gear of the third proximal rotatable drive shaft, wherein the ring gear is keyed to the inner housing; and at least one coupling bar having a proximal end connected to the inner housing, a boss on the proximal end to be in mechanical cooperation with the groove in the intermediate housing and a distal end connected to a distal coupling assembly, wherein the distal coupling assembly is configured to selectively connect with the end effector. Rotation of the third rotatable drive shaft of the handle assembly results in rotation of the third proximal drive shaft, and rotation of the third proximal drive shaft results in rotation of the ring gear, the inner housing, the at least one coupling bar and the distal coupling assembly to rotate the end effector relative to the adapter and about a longitudinal axis defined by the adapter.

The outer tube may be configured for endoscopic insertion into a target surgical site. The adapter housing may be inhibited from insertion into the target surgical site.

At least one of the first drive converter assembly, the second drive converter assembly and the drive transmitting assembly may be disposed in the adapter housing.

In an embodiment the end effector and the outer tube of the adapter define an endoscopic portion that is configured for endoscopic insertion into a target surgical site.

### BRIEF DESCRIPTION OF THE DRAWINGS

Embodiments of the present disclosure are described herein with reference to the accompanying drawings, wherein:
FIG. 1 is a perspective view, with parts separated, of a surgical device and adapter, in accordance with an embodiment of the present disclosure, illustrating a connection thereof with an end effector;
FIG. 2 is a perspective view of the surgical device of FIG. 1;
FIG. 3 is a perspective view, with parts separated, of the surgical device of FIGS. 1 and 2;
FIG. 4 is a perspective view of a battery for use in the surgical device of FIGS. 1-3;
FIG. 5 is a perspective view of the surgical device of FIGS. 1-3, with a housing thereof removed;
FIG. 6 is a perspective view of the connecting ends of each of the surgical device and the adapter, illustrating a connection therebetween;
FIG. 7 is a cross-sectional view of the surgical device of FIGS. 1-3, as taken through 7-7 of FIG. 2;
FIG. 8 is a cross-sectional view of the surgical device of FIGS. 1-3, as taken through 8-8 of FIG. 2;
FIG. 9 is a perspective view, with parts separated, of a trigger housing of the surgical device of FIGS. 1-3;
FIG. 10 is a perspective view of the adapter of FIG. 1;
FIG. 11 is a perspective view, with parts separated, of the adapter of FIGS. 1 and 10;
FIG. 11A is a perspective view of an alternate embodiment of an inner housing half;
FIG. 11B is a plan view of an alternate embodiment of a handle housing half;
FIG. 12 is a perspective view, with parts separated, of a drive coupling assembly of the adapter of FIGS. 1 and 10;
FIG. 13 is a perspective view, with parts separated, of a distal portion of the adapter of FIGS. 1 and 10;
FIG. 13A is a perspective view of an alternate embodiment of a rotation transmitting bar;
FIG. 13B is a perspective view of an alternate embodiment of an intermediate housing;
FIG. 14 is a cross-sectional view of the adapter of FIGS. 1 and 10, as taken through 14-14 of FIG. 10;
FIG. 15 is a cross-sectional view of the adapter of FIGS. 1 and 10, as taken through 15-15 of FIG. 10;
FIG. 16 is an enlarged view of the indicated area of detail of FIG. 14;
FIG. 16A is an enlarged view of an alternate embodiment of the indicated area of detail of FIG. 14 including the alternate embodiment of a rotation transmitting bar of FIG. 13A and the alternate embodiment of an intermediate housing of FIG. 13B;
FIG. 17 is an enlarged view of the indicated area of detail of FIG. 15;
FIG. 17A is an enlarged view of an alternate embodiment of the indicated area of detail of FIG. 15 including the alternate embodiment of an inner housing of FIG. 11A and the alternate embodiment of a handle housing half of FIG. 11B;
FIG. 18 is an enlarged view of the indicated area of detail of FIG. 14;
FIG. 19 is an enlarged view of the indicated area of detail of FIG. 15;
FIG. 20 is a perspective view, with parts separated, of a coupling cuff of the adapter of FIGS. 1 and 10;
FIG. 21 is a perspective view, with parts separated, of an exemplary end effector for use with the surgical device and the adapter of the present disclosure; and
FIG. 22 is a schematic illustration of the outputs to the LED's; selection of motor (to select clamping/cutting, rotation or articulation); and selection of the drive motors to perform a function selected.

### DETAILED DESCRIPTION OF EMBODIMENTS

Embodiments of the presently disclosed surgical devices, and adapter assemblies for surgical devices and/or handle assemblies are described in detail with reference to the drawings, in which like reference numerals designate identical or corresponding elements in each of the several views. As used herein the term "distal" refers to that portion of the adapter or surgical device, or component thereof, farther from the user, while the term "proximal" refers to that portion of the adapter or surgical device, or component thereof, closer to the user.

A surgical device, in accordance with an embodiment of the present disclosure, is generally designated as 100, and is in the form of a powered hand held electromechanical instrument configured for selective attachment thereto of a plurality of different end effectors that are each configured for actuation and manipulation by the powered hand held electromechanical surgical instrument.

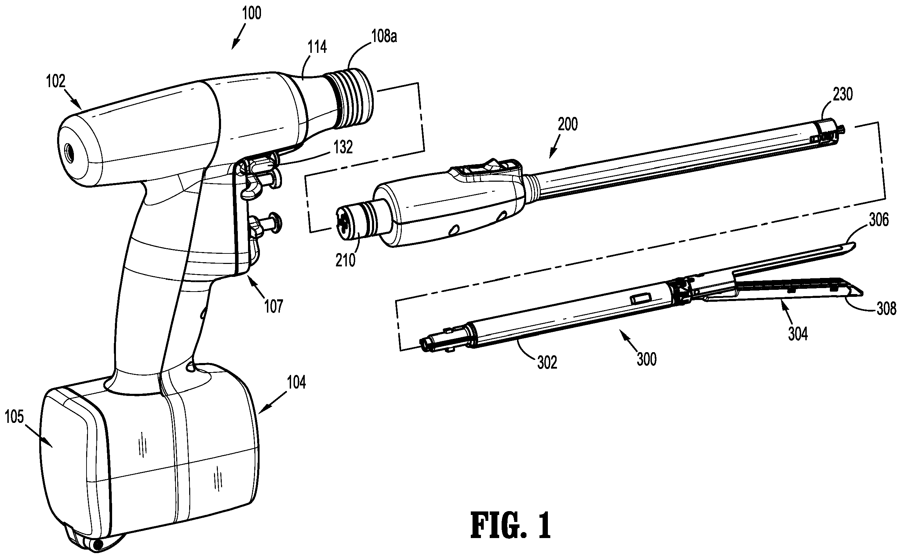

As illustrated in FIG. 1, surgical device 100 is configured for selective connection with an adapter 200, and, in turn, adapter 200 is configured for selective connection with an end effector or single use loading unit 300.

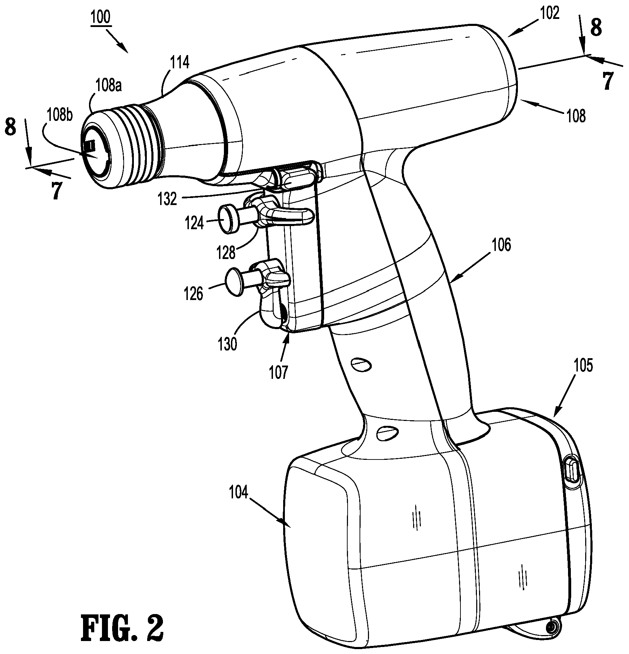

As illustrated in FIGS. 1-3, surgical device 100 includes a handle housing 102 having a lower housing portion 104, an intermediate housing portion 106 extending from and/or supported on lower housing portion 104, and an upper housing portion 108 extending from and/or supported on intermediate housing portion 106. Intermediate housing portion 106 and upper housing portion 108 are separated into a distal half-section 110a that is integrally formed with and extending from the lower portion 104, and a proximal half-section 110b connectable to distal half-section 110a by a plurality of fasteners. When joined, distal and proximal half-sections 110a, 110b define a handle housing 102 having a cavity 102a therein in which a circuit board 150 and a drive mechanism 160 is situated.

Distal and proximal half-sections 110a, 110b are divided along a plane that traverses a longitudinal axis "X" of upper housing portion 108, as seen in FIG. 1.

Handle housing 102 includes a gasket 112 extending completely around a rim of distal half-section and/or proximal half-section 110a, 110b and being interposed between distal half-section 110a and proximal half-section 110b. Gasket 112 seals the perimeter of distal half-section 110a and proximal half-section 110b. Gasket 112 functions to establish an air-tight seal between distal half-section 110a and proximal half-section 110b such that circuit board 150 and drive mechanism 160 are protected from sterilization and/or cleaning procedures.

In this manner, the cavity 102a of handle housing 102 is sealed along the perimeter of distal half-section 110a and proximal half-section 110b yet is configured to enable easier, more efficient assembly of circuit board 150 and a drive mechanism 160 in handle housing 102.

Intermediate housing portion 106 of handle housing 102 provides a housing in which circuit board 150 is situated. Circuit board 150 is configured to control the various operations of surgical device 100, as will be set forth in additional detail below.

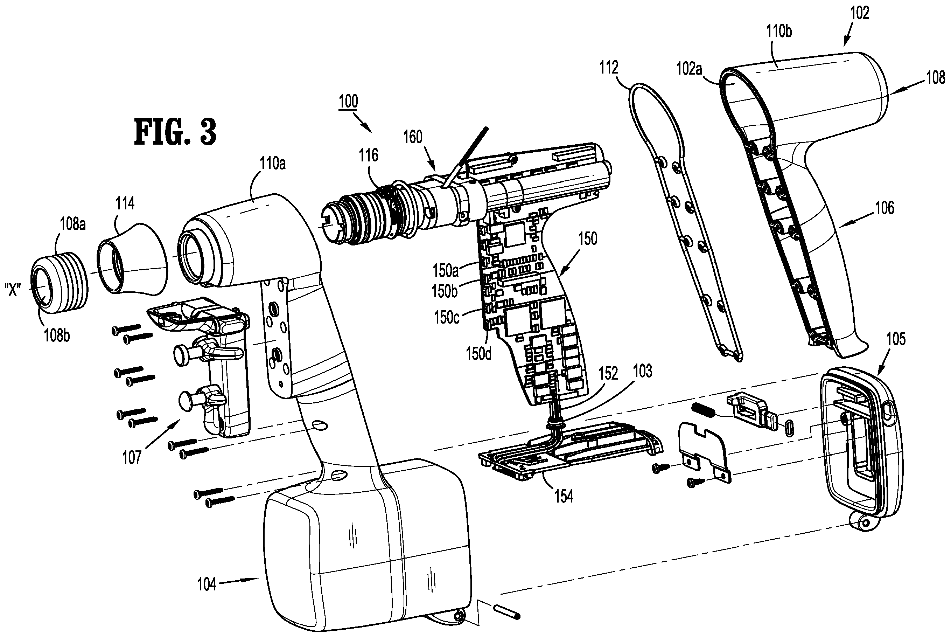

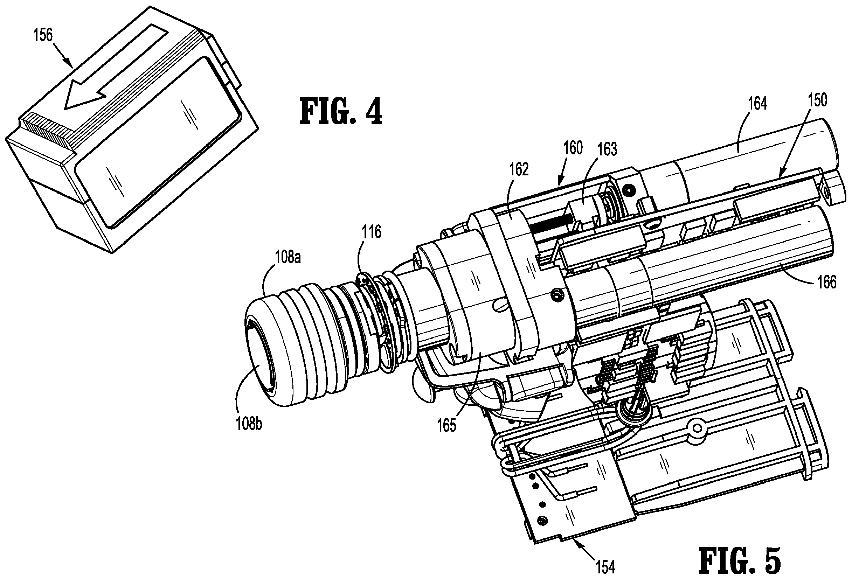

Lower housing portion 104 of surgical device 100 defines an aperture (not shown) formed in an upper surface thereof and which is located beneath or within intermediate housing portion 106. The aperture of lower housing portion 104 provides a passage through which wires 152 pass to electrically interconnect electrical components (a battery 156, as illustrated in FIG. 4, a circuit board 154, as illustrated in FIG. 3, etc.) situated in lower housing portion 104 with electrical components (circuit board 150, drive mechanism 160, etc.) situated in intermediate housing portion 106 and/or upper housing portion 108.

Handle housing 102 includes a gasket 103 disposed within the aperture of lower housing portion 104 (not shown) thereby plugging or sealing the aperture of lower housing portion 104 while allowing wires 152 to pass therethrough. Gasket 103 functions to establish an air-tight seal between lower housing portion 106 and intermediate housing portion 108 such that circuit board 150 and drive mechanism 160 are protected from sterilization and/or cleaning procedures.

As shown, lower housing portion 104 of handle housing 102 provides a housing in which a rechargeable battery 156, is removably situated. Battery 156 is configured to supply power to any of the electrical components of surgical device 100. Lower housing portion 104 defines a cavity (not shown) into which battery 156 is inserted. Lower housing portion 104 includes a door 105 pivotally connected thereto for closing cavity of lower housing portion 104 and retaining battery 156 therein.

With reference to FIGS. 3 and 5, distal half-section 110a of upper housing portion 108 defines a nose or connecting portion 108a. A nose cone 114 is supported on nose portion 108a of upper housing portion 108. Nose cone 114 is fabricated from a transparent material. An illumination member 116 is disposed within nose cone 114 such that illumination member 116 is visible therethrough. Illumination member 116 is in the form of a light emitting diode printed circuit board (LED PCB). Illumination member 116 is configured to illuminate multiple colors with a specific color pattern being associated with a unique discrete event.

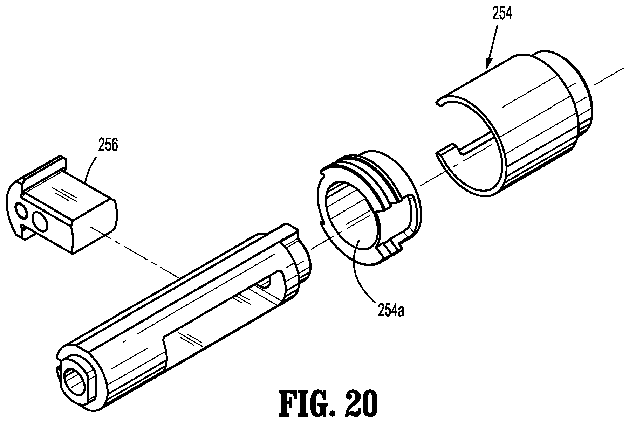

Upper housing portion 108 of handle housing 102 provides a housing in which drive mechanism 160 is situated. As illustrated in FIG. 5, drive mechanism 160 is configured to drive shafts and/or gear components in order to perform the various operations of surgical device 100. In particular, drive mechanism 160 is configured to drive shafts and/or gear components in order to selectively move tool assembly 304 of end effector 300 (see FIGS. 1 and 20) relative to proximal body portion 302 of end effector 300, to rotate end effector 300 about a longitudinal axis "X" (see FIG. 3) relative to handle housing 102, to move anvil assembly 306 relative to cartridge assembly 308 of end effector 300, and/or to fire a stapling and cutting cartridge within cartridge assembly 308 of end effector 300.

The drive mechanism 160 includes a selector gearbox assembly 162 that is located immediately proximal relative to adapter 200. Proximal to the selector gearbox assembly 162 is a function selection module 163 having a first motor 164 that functions to selectively move gear elements within the selector gearbox assembly 162 into engagement with an input drive component 165 having a second motor 166.

As illustrated in FIGS. 1-4, and as mentioned above, distal half-section 110a of upper housing portion 108 defines a connecting portion 108a configured to accept a corresponding drive coupling assembly 210 of adapter 200.

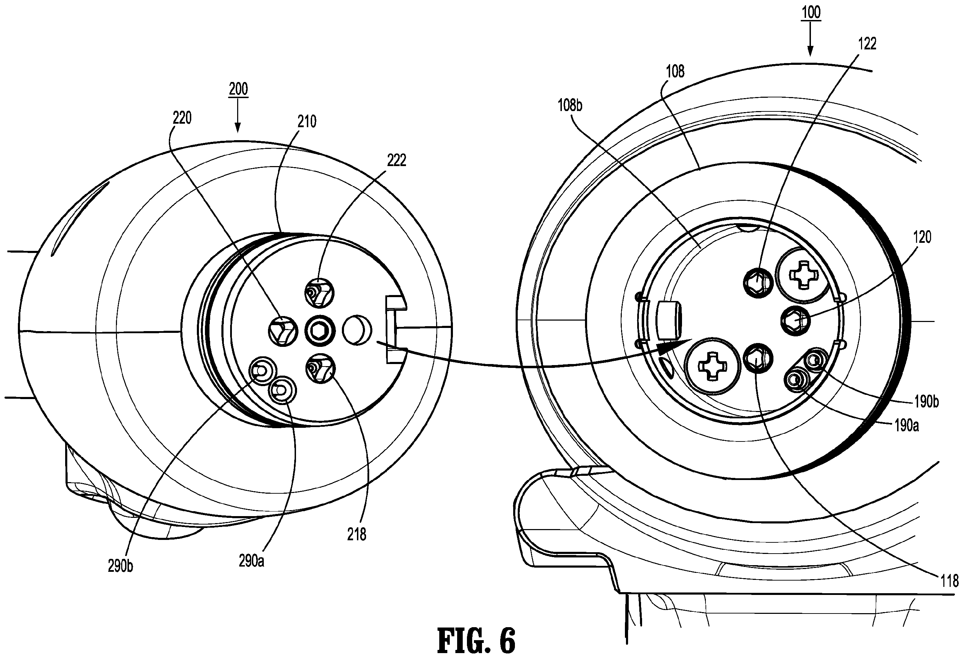

As illustrated in FIGS. 6-8, connecting portion 108a of surgical device 100 has a cylindrical recess 108b that receives a drive coupling assembly 210 of adapter 200 when adapter 200 is mated to surgical device 100. Connecting portion 108a houses three rotatable drive connectors 118, 120, 122.

When adapter 200 is mated to surgical device 100, each of rotatable drive connectors 118, 120, 122 of surgical device 100 couples with a corresponding rotatable connector sleeve 218, 220, 222 of adapter 200. (see FIG. 6). In this regard, the interface between corresponding first drive connector 118 and first connector sleeve 218, the interface between corresponding second drive connector 120 and second connector sleeve 220, and the interface between corresponding third drive connector 122 and third connector sleeve 222 are keyed such that rotation of each of drive connectors 118, 120, 122 of surgical device 100 causes a corresponding rotation of the corresponding connector sleeve 218, 220, 222 of adapter 200.

The mating of drive connectors 118, 120, 122 of surgical device 100 with connector sleeves 218, 220, 222 of adapter 200 allows rotational forces to be independently transmitted via each of the three respective connector interfaces. The drive connectors 118, 120, 122 of surgical device 100 are configured to be independently rotated by drive mechanism 160. In this regard, the function selection module 163 of drive mechanism 160 selects which drive connector or connectors 118, 120, 122 of surgical device 100 is to be driven by the input drive component 165 of drive mechanism 160.

Since each of drive connectors 118, 120, 122 of surgical device 100 has a keyed and/or substantially non-rotatable interface with respective connector sleeves 218, 220, 222 of adapter 200, when adapter 200 is coupled to surgical device 100, rotational force(s) are selectively transferred from drive mechanism 160 of surgical device 100 to adapter 200.

The selective rotation of drive connector(s) 118, 120 and/or 122 of surgical device 100 allows surgical device 100 to selectively actuate different functions of end effector 300. As will be discussed in greater detail below, selective and independent rotation of first drive connector 118 of surgical device 100 corresponds to the selective and independent opening and closing of tool assembly 304 of end effector 300, and driving of a stapling/cutting component of tool assembly 304 of end effector 300. Also, the selective and independent rotation of second drive connector 120 of surgical device 100 corresponds to the selective and independent articulation of tool assembly 304 of end effector 300 transverse to longitudinal axis "X" (see FIG. 3). Additionally, the selective and independent rotation of third drive connector 122 of surgical device 100 corresponds to the selective and independent rotation of end effector 300 about longitudinal axis "X" (see FIG. 3) relative to handle housing 102 of surgical device 100.

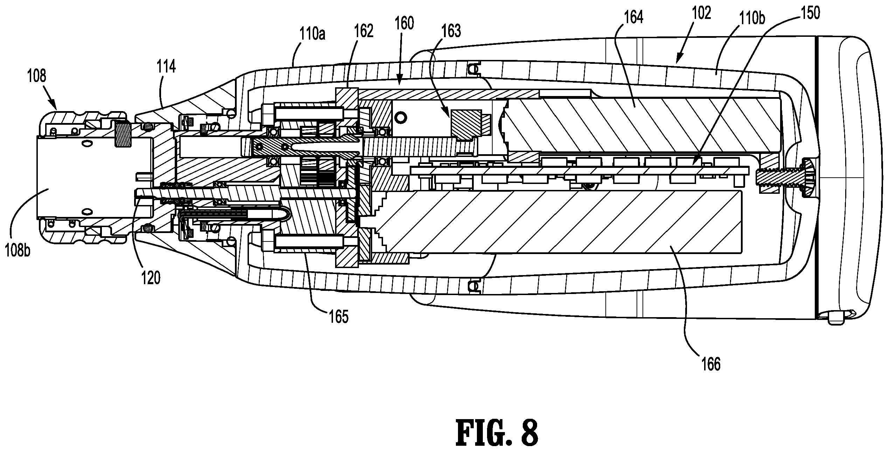

As mentioned above and as illustrated in FIGS. 5 and 8, drive mechanism 160 includes a selector gearbox assembly 162; a function selection module 163, located proximal to the selector gearbox assembly 162, that functions to selectively move gear elements within the selector gearbox assembly 162 into engagement with second motor 166. Thus, drive mechanism 160 selectively drives one of drive connectors 118, 120, 122 of surgical device 100 at a given time.

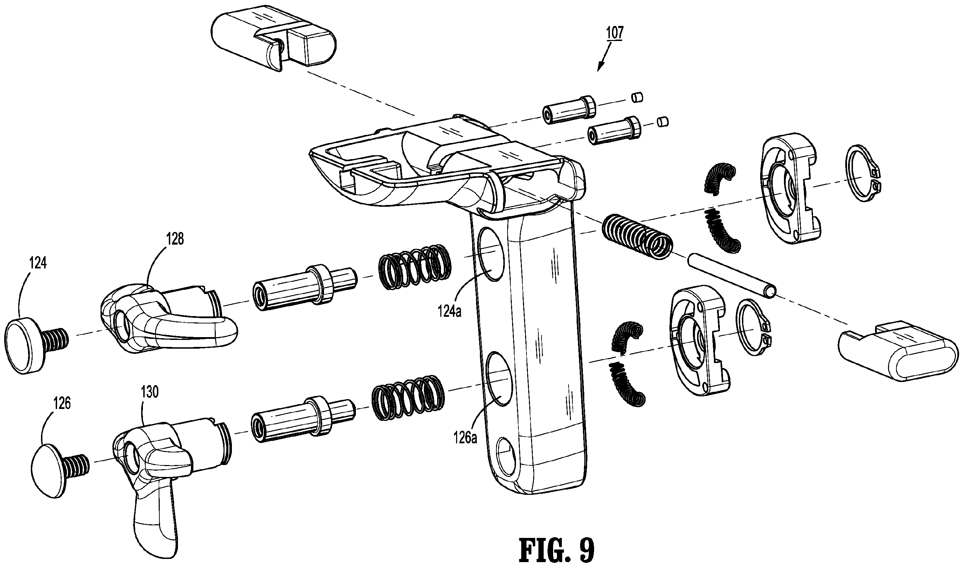

As illustrated in FIGS. 1-3 and FIG. 9, handle housing 102 supports a trigger housing 107 on a distal surface or side of intermediate housing portion 108. Trigger housing 107, in cooperation with intermediate housing portion 108, supports a pair of finger-actuated control buttons 124, 126 and rocker devices 128, 130. In particular, trigger housing 107 defines an upper aperture 124a for slidably receiving a first control button 124, and a lower aperture 126b for slidably receiving a second control button 126.

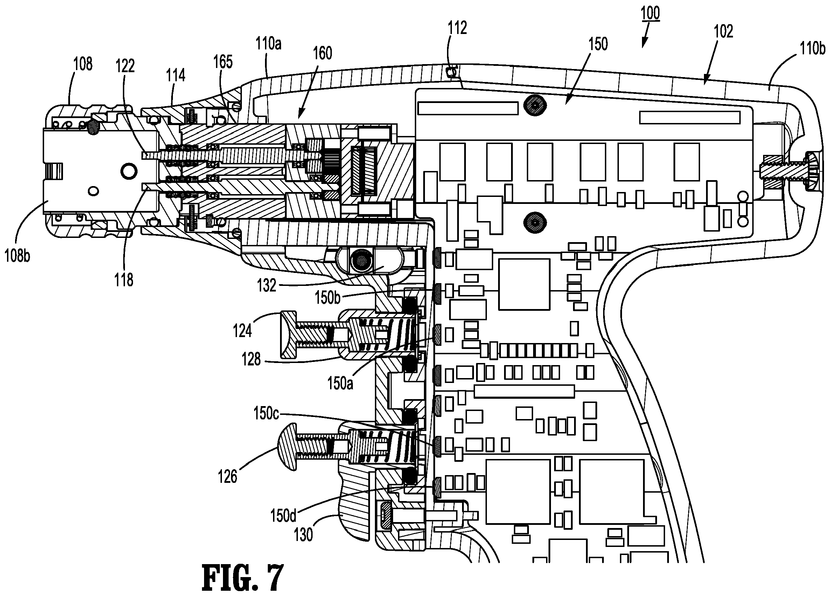

Each one of the control buttons 124, 126 and rocker devices 128, 130 includes a respective magnet (not shown) that is moved by the actuation of an operator. In addition, circuit board 150 includes, for each one of the control buttons 124, 126 and rocker devices 128, 130, respective Hall-effect switches 150a-150d that are actuated by the movement of the magnets in the control buttons 124, 126 and rocker devices 128, 130. In particular, located immediately proximal to the control button 124 is a first Hall-effect switch 150a (see FIGS. 3 and 7) that is actuated upon the movement of a magnet within the control button 124 upon the operator actuating control button 124. The actuation of first Hall-effect switch 150a, corresponding to control button 124, causes circuit board 150 to provide appropriate signals to function selection module 163 and input drive component 165 of the drive mechanism 160 to close a tool assembly 304 of end effector 300 and/or to fire a stapling/cutting cartridge within tool assembly 304 of end effector 300.

Also, located immediately proximal to rocker device 128 is a second Hall-effect switch 150b (see FIGS. 3 and 7) that is actuated upon the movement of a magnet (not shown) within rocker device 128 upon the operator actuating rocker device 128. The actuation of second Hall-effect switch 150b, corresponding to rocker device 128, causes circuit board 150 to provide appropriate signals to function selection module 163 and input drive component 165 of drive mechanism 160 to articulate tool assembly 304 relative to body portion 302 of end effector 300. Advantageously, movement of rocker device 128 in a first direction causes tool assembly 304 to articulate relative to body portion 302 in a first direction, while movement of rocker device 128 in an opposite, e.g., second, direction causes tool assembly 304 to articulate relative to body portion 302 in an opposite, e.g., second, direction.

Furthermore, located immediately proximal to control button 126 is a third Hall-effect switch 150c (see FIGS. 3 and 7) that is actuated upon the movement of a magnet (not shown) within control button 126 upon the operator actuating control button 126. The actuation of third Hall-effect switch 150c, corresponding to control button 126, causes circuit board 150 to provide appropriate signals to function selection module 163 and input drive component 165 of drive mechanism 160 to open tool assembly 304 of end effector 300.

In addition, located immediately proximal to rocker device 130 is a fourth Hall-effect switch 150d (see FIGS. 3 and 7) that is actuated upon the movement of a magnet (not shown) within rocker device 130 upon the operator actuating rocker device 130. The actuation of fourth Hall-effect switch 150d, corresponding to rocker device 130, causes circuit board 150 to provide appropriate signals to function selection module 163 and input drive component 165 of drive mechanism 160 to rotate end effector 300 relative to handle housing 102 surgical device 100. Specifically, movement of rocker device 130 in a first direction causes end effector 300 to rotate relative to handle housing 102 in a first direction, while movement of rocker device 130 in an opposite, e.g., second, direction causes end effector 300 to rotate relative to handle housing 102 in an opposite, e.g., second, direction.

As seen in FIGS. 1-3, surgical device 100 includes a fire button or safety switch 132 supported between intermediate housing portion 108 and upper housing portion, and situated above trigger housing 107. In use, tool assembly 304 of end effector 300 is actuated between opened and closed conditions as needed and/or desired. In order to fire end effector 300, to expel fasteners therefrom when tool assembly 304 of end effector 300 is in a closed condition, safety switch 132 is depressed thereby instructing surgical device 100 that end effector 300 is ready to expel fasteners therefrom.

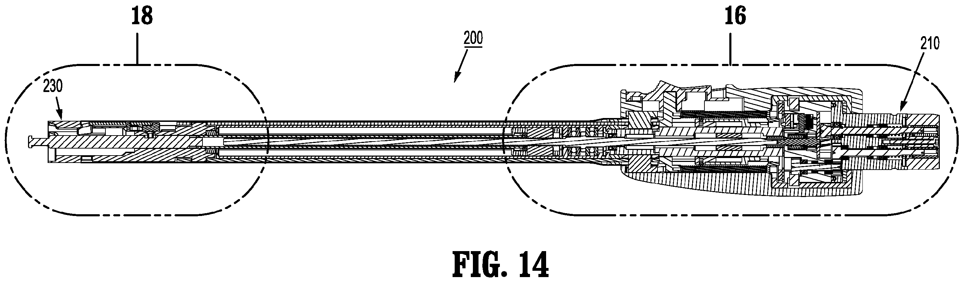

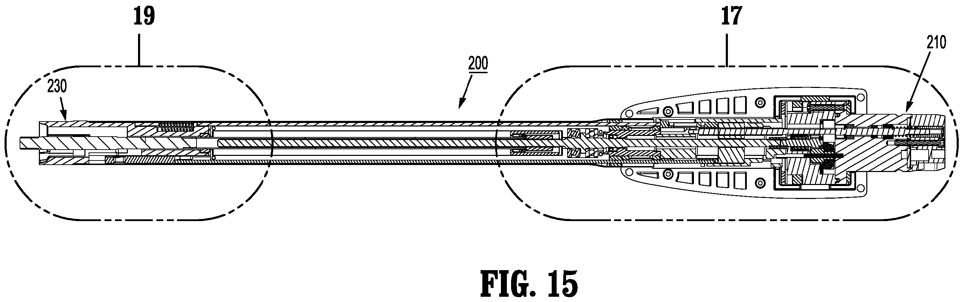

As illustrated in FIGS. 1 and 10-20, surgical device 100 is configured for selective connection with adapter 200, and, in turn, adapter 200 is configured for selective connection with end effector 300.

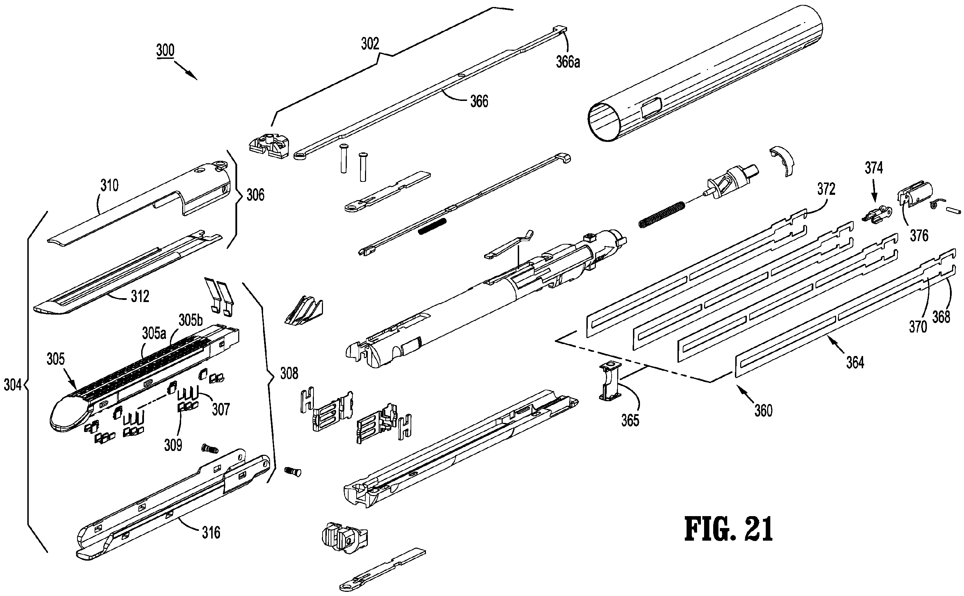

Adapter 200 is configured to convert a rotation of either of drive connectors 120 and 122 of surgical device 100 into axial translation useful for operating a drive assembly 360 and an articulation link 366 of end effector 300, as illustrated in FIG. 21 and as will be discussed in greater detail below.

Adapter 200 includes a first drive transmitting/converting assembly for interconnecting third rotatable drive connector 122 of surgical device 100 and a first axially translatable drive member of end effector 300, wherein the first drive transmitting/converting assembly converts and transmits a rotation of third rotatable drive connector 122 of surgical device 100 to an axial translation of the first axially translatable drive assembly 360 of end effector 300 for firing.

Adapter 200 includes a second drive transmitting/converting assembly for interconnecting second rotatable drive connector 120 of surgical device 100 and a second axially translatable drive member of end effector 300, wherein the second drive transmitting/converting assembly converts and transmits a rotation of second rotatable drive connector 120 of surgical device 100 to an axial translation of articulation link 366 of end effector 300 for articulation.

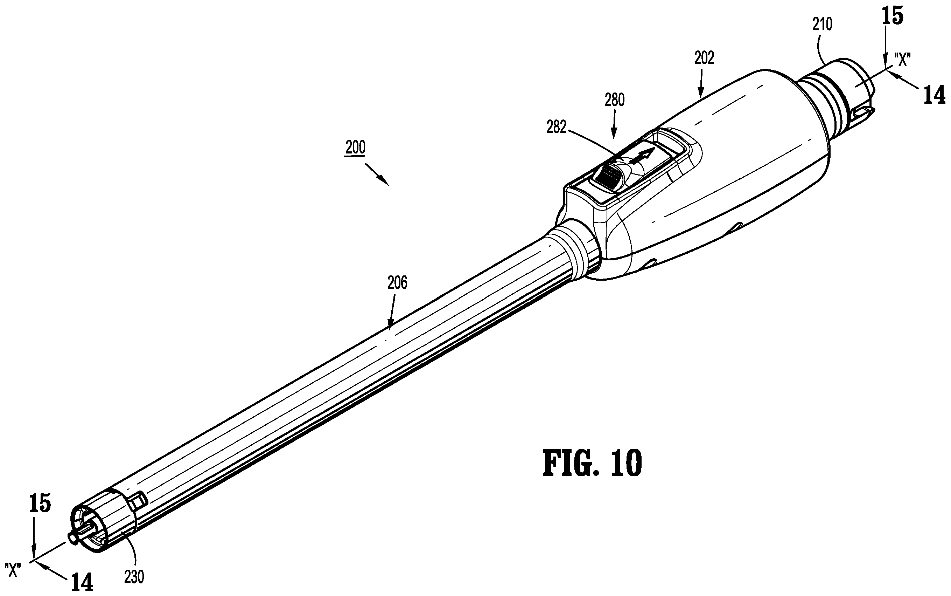

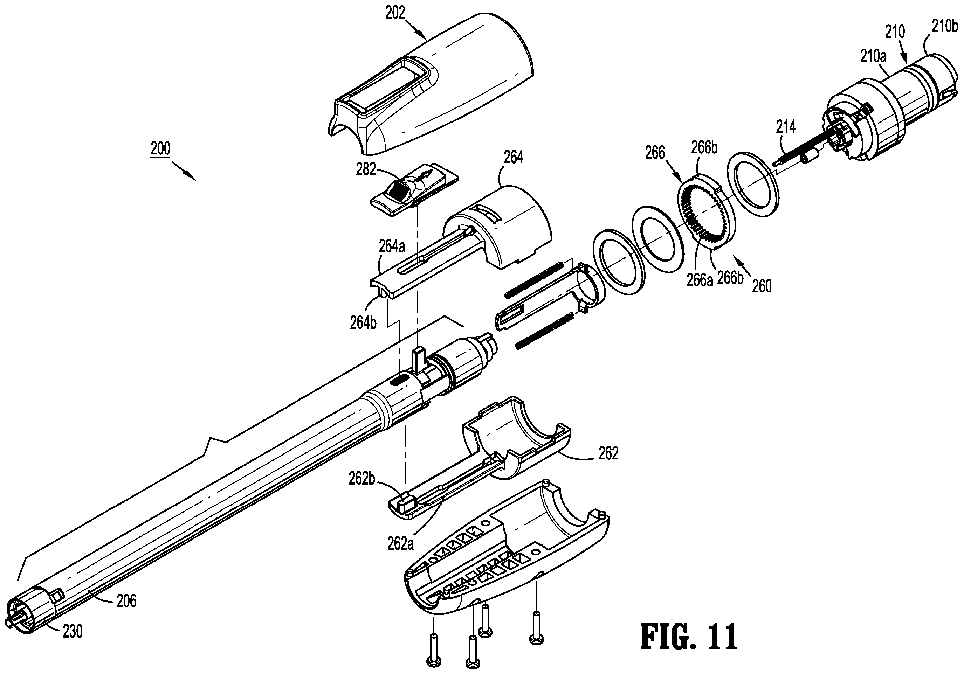

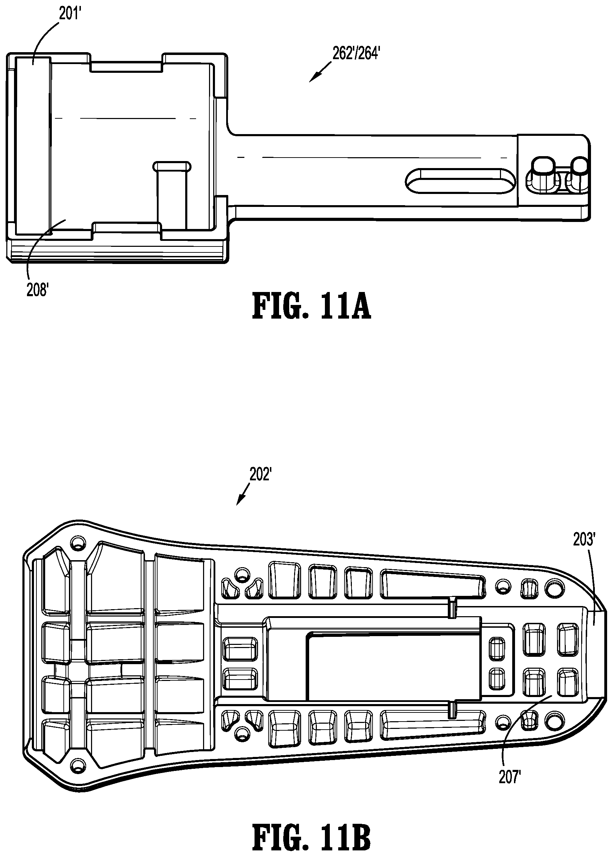

Turning now to FIGS. 10 and 11, adapter 200 includes a knob housing 202 and an outer tube 206 extending from a distal end of knob housing 202. Knob housing 202 and outer tube 206 are configured and dimensioned to house the components of adapter 200. Outer tube 206 is dimensioned for endoscopic insertion, in particular, that outer tube is passable through a typical trocar port, cannula or the like. Knob housing 202 is dimensioned to not enter the trocar port, cannula of the like.

Knob housing 202 is configured and adapted to connect to connecting portion 108a of upper housing portion 108 of distal half-section 110a of surgical device 100.

As seen in FIGS. 10-12, adapter 200 includes a surgical device drive coupling assembly 210 at a proximal end thereof and to an end effector coupling assembly 230 at a distal end thereof. Drive coupling assembly 210 includes a distal drive coupling housing 210a and a proximal drive coupling housing 210b rotatably supported, at least partially, in knob housing 202. Drive coupling assembly 210 rotatably supports a first rotatable proximal drive shaft 212, a second rotatable proximal drive shaft 214, and a third rotatable proximal drive shaft 216 therein.

Proximal drive coupling housing 210b is configured to rotatably support first, second and third connector sleeves 218, 220 and 222, respectively. Each of connector sleeves 218, 220, 222 is configured to mate with respective first, second and third drive connectors 118, 120, 122 of surgical device 100, as described above. Each of connector sleeves 218, 220, 222 is further configured to mate with a proximal end of respective first, second and third proximal drive shafts 212, 214, 216.

Proximal drive coupling assembly 210 includes a first, a second and a third biasing member 224, 226 and 228 disposed distally of respective first, second and third connector sleeves 218, 220, 222. Each of biasing members 224, 226 and 228 is disposed about respective first, second and third rotatable proximal drive shaft 212, 214 and 216. Biasing members 224, 226 and 228 act on respective connector sleeves 218, 220 and 222 to help maintain connector sleeves 218, 220 and 222 engaged with the distal end of respective drive rotatable drive connectors 118, 120, 122 of surgical device 100 when adapter 200 is connected to surgical device 100.

In particular, first, second and third biasing members 224, 226 and 228 function to bias respective connector sleeves 218, 220 and 222 in a proximal direction. In this manner, during assembly of adapter 200 to surgical device 100, if first, second and or third connector sleeves 218, 220 and/or 222 is/are misaligned with the drive connectors 118, 120, 122 of surgical device 100, first, second and/or third biasing member(s) 224, 226 and/or 228 are compressed. Thus, when drive mechanism 160 of surgical device 100 is engaged, drive connectors 118, 120, 122 of surgical device 100 will rotate and first, second and/or third biasing member(s) 224, 226 and/or 228 will cause respective first, second and/or third connector sleeve(s) 218, 220 and/or 222 to slide back proximally, effectively coupling drive connectors 118, 120, 122 of surgical device 100 to first, second and/or third proximal drive shaft(s) 212, 214 and 216 of proximal drive coupling assembly 210.

Upon calibration of surgical device 100, each of drive connectors 118, 120, 122 of surgical device 100 is rotated and the bias on connector sleeve(s) 218, 220 and 222 properly seats connector sleeve(s) 218, 220 and 222 over the respective drive connectors 118, 120, 122 of surgical device 100 when the proper alignment is reached.

Adapter 200 includes a first, a second and a third drive transmitting/converting assembly 240, 250, 260, respectively, disposed within handle housing 202 and outer tube 206. Each drive transmitting/converting assembly 240, 250, 260 is configured and adapted to transmit or convert a rotation of a first, second and third drive connector 118, 120, 122 of surgical device 100 into axial translation of drive tube 246 and drive bar 258 of adapter 200, to effectuate closing, opening, articulating and firing of end effector 300; or a rotation of ring gear 266 of adapter 200, to effectuate rotation of adapter 200.

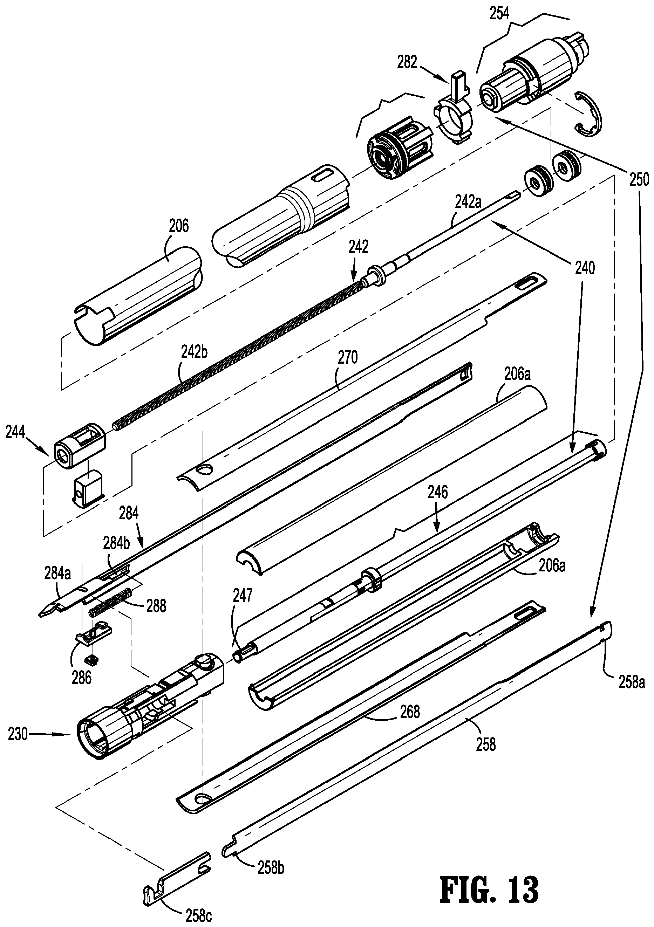

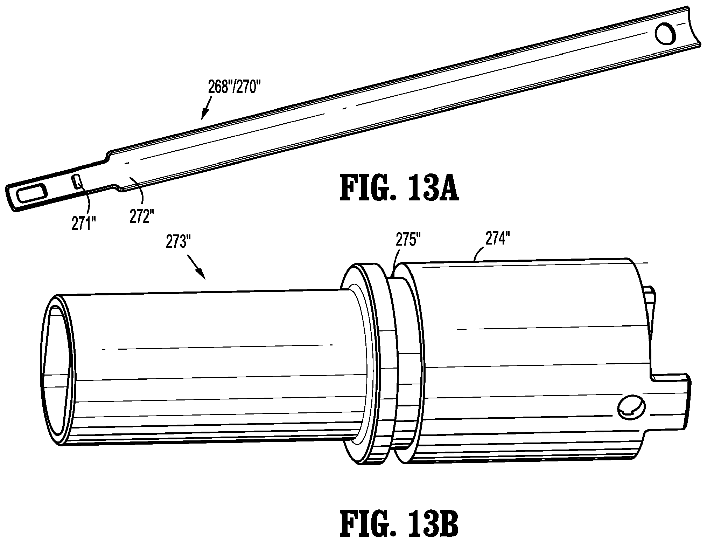

As seen in FIGS. 13-19, first drive transmitting/converting assembly 240 includes a first distal drive shaft 242 rotatably supported within housing 202 and outer tube 206. A proximal end portion 242a of first distal drive shaft 242 is keyed to a spur gear 242c which is configured for connection to a spur gear 212a keyed to first rotatable proximal drive shaft 212, via a compound gear 243. First distal drive shaft 242 further includes a distal end portion 242b having a threaded outer profile or surface.

First drive transmitting/converting assembly 240 further includes a drive coupling nut 244 rotatably coupled to threaded distal end portion 242b of first distal drive shaft 242, and which is slidably disposed within outer tube 206. Drive coupling nut 244 is keyed to an inner housing tube 206a of outer tube 206 so as to be prevented from rotation as first distal drive shaft 242 is rotated. In this manner, as first distal drive shaft 242 is rotated, drive coupling nut 244 is translated through and/or along inner housing tube 206a of outer tube 206.

First drive transmitting/converting assembly 240 further includes a drive tube 246 surrounding first distal drive shaft 242 and having a proximal end portion connected to drive coupling nut 244 and a distal end portion extending beyond a distal end of first distal drive shaft 242. The distal end portion of drive tube 246 supports a connection member 247 (see FIG. 13) configured and dimensioned for selective engagement with drive member 374 of drive assembly 360 of end effector 300.

In operation, as first rotatable proximal drive shaft 212 is rotated, due to a rotation of first connector sleeve 218, as a result of the rotation of the first respective drive connector 118 of surgical device 100, spur gear 212a of first rotatable proximal drive shaft 212 engages first gear 243a of compound gear 243 causing compound gear 243 to rotate. As compound gear 243 rotates, a second gear 243b of compound gear 243 is rotated and thus causes spur gear 242c that is keyed to first distal drive shaft 242, that is engaged therewith, to also rotate thereby causing first distal drive shaft 242 to rotate. As first distal drive shaft 242 is rotated, drive coupling nut 244 is caused to be translated axially along first distal drive shaft 242.

As drive coupling nut 244 is caused to be translated axially along first distal drive shaft 242, drive tube 246 is caused to be translated axially relative to inner housing tube 206a of outer tube 206. As drive tube 246 is translated axially, with connection member 247 connected thereto and connected to a drive member 374 of drive assembly 360 of end effector 300, drive tube 246 causes concomitant axial translation of drive member 374 of end effector 300 to effectuate a closure of tool assembly 304 and a firing of tool assembly 304 of end effector 300.

With reference to FIGS. 13-19, second drive converter assembly 250 of adapter 200 includes second rotatable proximal drive shaft 214 rotatably supported within drive coupling assembly 210. Second rotatable proximal drive shaft 214 includes a non-circular or shaped proximal end portion 214a configured for connection with second connector 220 which is connected to respective second connector 120 of surgical device 100. Second rotatable proximal drive shaft 214 further includes a distal end portion 214b having a threaded outer profile or surface.

As illustrated in FIG. 20, second drive converter assembly 250 further includes a coupling cuff 254 rotatably and translatably supported within an annular race or recess formed in knob housing 202. Coupling cuff 254 defines a lumen 254a therethrough, and an annular race or recess formed in a surface of lumen 254a. Second drive converter assembly 250 further includes a coupling slider 256 extending across lumen 254a of coupling cuff 254 and slidably disposed within the race of coupling cuff 254. Coupling slider 256 is threadably connected to threaded distal end portion 214b of second rotatable proximal drive shaft 214. As so configured, coupling cuff 254 can rotate about second rotatable proximal drive shaft 214, thereby maintaining a radial position of second rotatable proximal drive shaft 214 relative to first rotatable proximal drive shaft 242.

Second rotatable proximal drive shaft 214 defines an axis of rotation, and coupling cuff 254 defines an axis of rotation that is spaced a radial distance from the axis of rotation of second rotatable proximal drive shaft 214. Coupling slider 256 defines an axis of rotation that is coincident with the axis of rotation of coupling cuff 254.

Second drive converter assembly 250 further includes a drive bar 258 translatably supported for axial translation through outer tube 206. Drive bar 258 includes a proximal end portion 258a coupled to coupling cuff 254, and a distal end portion 258b defining a coupling hook 258c configured and dimensioned for selective engagement with hooked proximal end 366a of articulation link 366 of end effector 300. (see FIG. 21).

In operation, as illustrated in FIGS. 10-19, as drive shaft 214 is rotated due to a rotation of second connector sleeve 220, as a result of the rotation of the second drive connector 120 of surgical device 100, coupling slider 256 is caused to be translated axially along threaded distal portion 214b of second rotatable proximal drive shaft 214, which in turn causes coupling cuff 254 to be translated axially relative to knob housing 202. As coupling cuff 254 is translated axially, drive bar 258 is caused to be translated axially. Accordingly, as drive bar 258 is translated axially, with hook 258c thereof connected to hooked proximal end 366a of articulation link 366 of end effector 300 (see FIG. 21), drive bar 258 causes concomitant axial translation of articulation link 366 of end effector 300 to effectuate an articulation of tool assembly 304.

As seen in FIGS. 10-19 and as mentioned above, adapter 200 includes a third drive transmitting/converting assembly 260 supported in knob housing 202. Third drive transmitting/converting assembly 260 includes first and second rotation housing half-sections 262, 264 rotatably supported in knob housing 202, respectively, and an internal rotation ring gear 266 supported and interposed between first and second rotation housing half-sections 262, 264. Each of first and second rotation housing half-sections 262, 264 includes an arm 262a, 264b extending distally therefrom and which are parallel to one another and spaced a transverse distance from one another. Each arm 262a, 264a includes a boss 262b, 264b extending radially inward near a distal end thereof.

Third drive transmitting/converting assembly 260 further includes a pair of rotation transmitting bars 268, 270, each, connected at a proximal end thereof to bosses 262b, 264b of arms 262a, 264a, and at a distal end thereof to a distal coupling assembly 230 supported at a distal end of outer tube 206.

Third drive transmitting/converting assembly 260 includes a ring gear 266 defining an internal array of gear teeth 266a. Ring gear 266 includes a pair of diametrically opposed, radially extending protrusions 266b projecting form an outer edge thereof. Protrusions 266b are disposed within recesses 262c, 264c defined in an inner surface of first and second rotation housing half-sections 262, 264, such that rotation of ring gear 266 results in rotation of first and second rotation housing half-sections 262, 264.

Third drive transmitting/converting assembly 260 further includes third rotatable proximal drive shaft 216 rotatably supported within housing 202 and outer tube 206. A proximal end portion of third rotatable proximal drive shaft 216 is keyed to third connector 222 of adapter 200. Third rotatable proximal drive shaft 216 includes a spur gear 216a keyed to a distal end thereof. A gear set 274 inter-engages spur gear 216a of third rotatable proximal drive shaft 216 to gear teeth 266a of ring gear 266. Gear set 274 includes a first gear 274a engaged with spur gear 216a of third rotatable proximal drive shaft 216, and a second gear 274b engaged with gear teeth 266a of ring gear 266.

In operation, as illustrated in FIGS. 10-19, as third rotatable proximal drive shaft 216 is rotated, due to a rotation of third connector sleeve 222, as a result of the rotation of the third respective drive connector 122 of surgical device 100, spur gear 216a of third rotatable proximal drive shaft 216 engages first gear 272a of gear set 274 causing gear set 274 to rotate. As gear set 274 rotates, second gear 274b of gear set 274 is rotated and thus causes ring gear 266 to also rotate thereby causing first and second rotation housing half-sections 262, 264 to rotate. As first and second rotation housing half-sections 262, 264 are rotated, rotation transmitting bars 268, 270, and distal coupling assembly 230 connected thereto, are caused to be rotated about longitudinal axis "X" of adapter 200. As distal coupling 230 is rotated, end effector 300, that is connected to distal coupling assembly 230, is also caused to be rotated about a longitudinal axis of adapter 200.

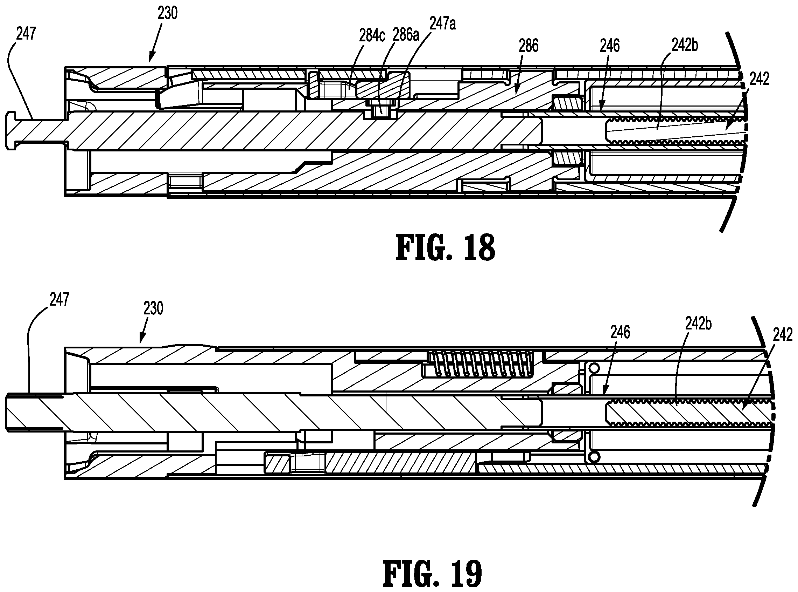

With reference to FIGS. 10, 11, 13 and 18, adapter 200 further includes a lock mechanism 280 for fixing the axial position and radial orientation of drive tube 246 for the connection and disconnection of end effector 300 thereto. Lock mechanism 280 includes a button 282 slidably supported on knob housing 202. Lock button 282 is connected to an actuation bar 284 that extends longitudinally through outer tube 206. Actuation bar 284 is interposed between outer tube 206 and inner housing tube 206a. Actuation bar 284 moves upon a movement of lock button 282. Actuation bar 284 includes a distal portion 284a defining a window 284b therein. As seen in FIG. 18, a distal end of window 284b defines a cam surface 284c.

As illustrated in FIGS. 13 and 18, lock mechanism 280 further includes a lock out 286 supported on distal coupling assembly 230 at a location in registration with window 284b of distal portion 284a of actuation bar 284. Lock out 286 includes a tab 286a extending toward connection member 247 of drive tube 246. Tab 286a of lock out 286 is configured and dimensioned to selectively engage a cut-out 247a formed in connection member 247 of drive tube 246. Lock mechanism 280 further includes a biasing member 288 tending to maintain lock out 286 and tab 286a thereof spaced away from cut-out 247a formed in connection member 247 of drive tube 246.

In operation, in order to lock the position and/or orientation of drive tube 246, a user moves lock button 282 from a distal position to a proximal position, thereby causing cam surface 284c of actuation bar 284 to engage lock arm 286 and urge lock out 286 toward drive tube 246, against the bias of biasing member 288, such that tab 286a of lock out 286 is received in cut-out 247a formed in connection member 247 of drive tube 246.

In this manner, drive tube 246 is prevented from distal and/or proximal movement. When lock button 282 is moved from the proximal position to the distal position, cam surface 284c is disengaged from lock out 286 thereby allowing biasing member 288 to urge lock out 286 and tab 286a thereof out of cut-out 247a formed in connection member 247 of drive tube 246.

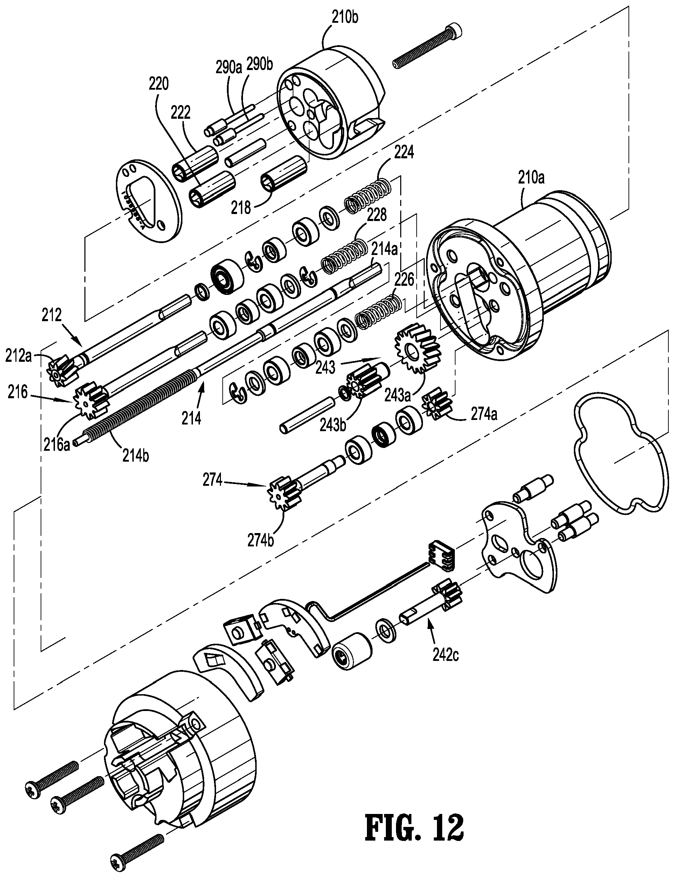

As seen in FIGS. 6 and 12, adapter 200 includes a pair of electrical contact pins 290a, 290b for electrical connection to a corresponding electrical plug 190a, 190b disposed in connecting portion 108a of surgical device 100. Electrical contacts 290a, 290b serve to allow for calibration and communication of necessary life-cycle information to circuit board 150 of surgical device 100 via electrical plugs 190a, 190b that are electrically connected to circuit board 150. Adapter 200 further includes a circuit board 292 supported in knob housing 202 and which is in electrical communication with electrical contact pins 290a, 290b.

When a button is activated by the user, the software checks predefined conditions. If conditions are met, the software controls the motors and delivers mechanical drive to the attached surgical stapler, which can then open, close, rotate, articulate or fire depending on the function of the pressed button. The software also provides feedback to the user by turning colored lights on or off in a defined manner to indicate the status of surgical device 100, adapter 200 and/or end effector 300.

A high level electrical architectural view of the system is displayed below in Schematic "A" and shows the connections to the various hardware and software interfaces. Inputs from presses of buttons 124, 126 and from motor encoders of the drive shaft are shown on the left side of Schematic "A". The microcontroller contains the device software that operates surgical device 100, adapter 200 and/or end effector 300. The microcontroller receives inputs from and sends outputs to a MicroLAN, an Ultra ID chip, a Battery ID chip, and Adaptor ID chips. The MicroLAN, the Ultra ID chip, the Battery ID chip, and the Adaptor ID chips control surgical device 100, adapter 200 and/or end effector 300 as follows:

| | |
|---|---|
| MicroLAN | - Serial 1-wire bus communication to read/write system component ID information. |
| Ultra ID chip | - identifies surgical device 100 and records usage information. |
| Battery ID chip | - identifies the Battery 156 and records usage information. |
| Adaptor ID chip | - identifies the type of adapter 200, records the presence of an end effector 300, and records usage information. |

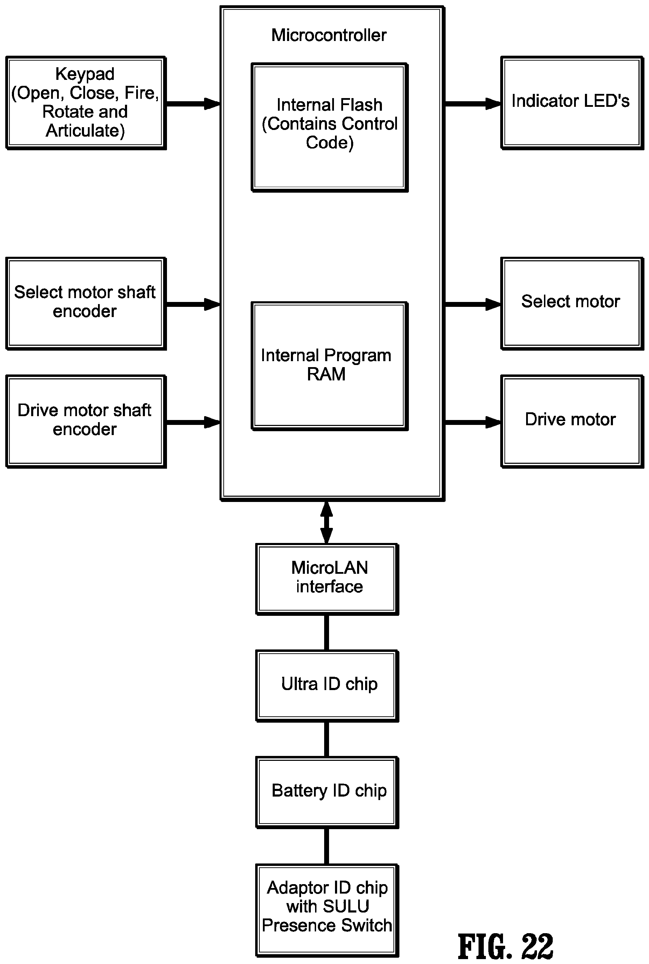

The right side of the schematic illustrated in FIG. 22 indicates outputs to the LED's; selection of motor (to select clamping/cutting, rotation or articulation); and selection of the drive motors to perform the function selected.

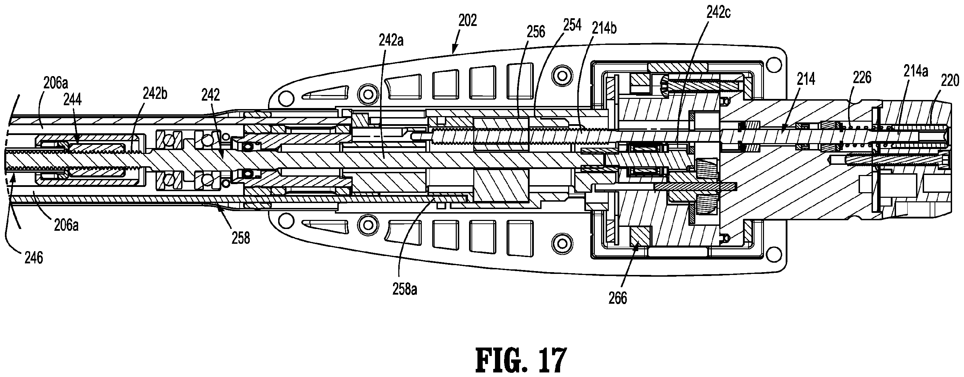

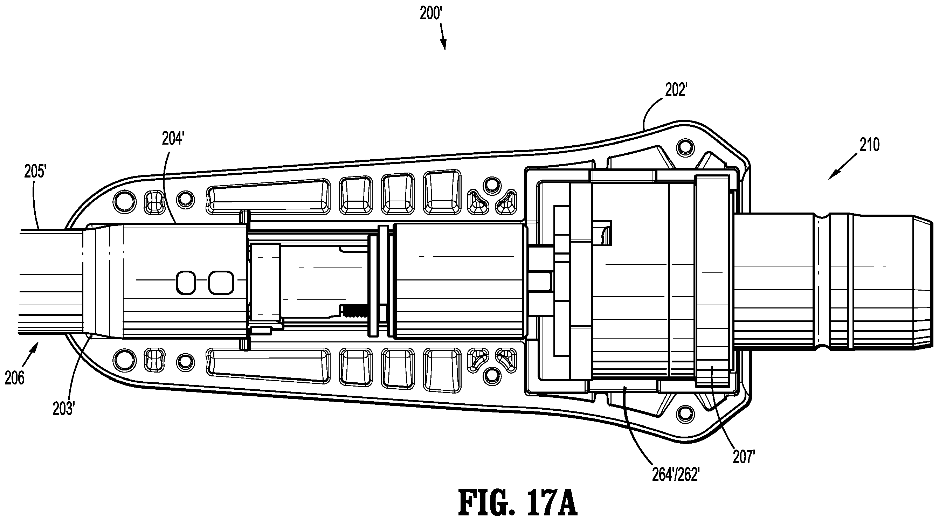

Referring to FIGS. 11A, 11B, and 17A, another embodiment of an adapter 200 provided in accordance with the present disclosure is shown and generally designated as 200'. Adapter 200' is configured similar to adapter 200 and therefore, in the interest of brevity, only the additional or alternate components thereof are described hereinafter.

Adapter 200' may include an alternate knob housing 202' (see FIG. 11B). Alternate knob housing 202' is configured similar to knob housing 202 as detailed above, and therefore, in the interest of brevity, only the additional or alternate features thereof are described hereinafter. Alternate knob housing 202' includes a longitudinal bore 207' configured to accept outer tube 206. Outer tube 206 includes a larger proximal diameter 204' tapering distally to a smaller distal diameter 205'. Longitudinal bore 207' of alternate knob housing 202' is configured to accept the larger proximal diameter 204' of outer tube 206 such that larger proximal diameter 204' is rotatably supported by longitudinal bore 207'. Housing 202' includes a flange 203' extending radially inward from the distal end of longitudinal bore 207'. Flange 203' is configured to receive the smaller distal diameter 205' of outer tube 206 such that smaller diameter 205' of outer tube 206 is supported by flange 203'. Flange 203' extends from knob housing 202' at a location such that the larger proximal diameter 205' of outer tube 206 is disposed within longitudinal bore 207' and is prevented from translating distally past flange 203'.

Adapter 200' may further include first and second rotation housing half sections 262' and 264'. First and second rotation housing half sections 262' and 264' are configured similarly to first and second rotation half sections 262 and 264 of above, and therefore, in the interest of brevity, only the additional or alternate features thereof are described hereinafter.

First and second rotation housing half sections 262' and 264' each define an inner wall surface 208', wherein an annular groove 201' (FIG. 11A) is defined in the proximal end of inner wall surface 208'. Annular groove 201' is configured to receive drive coupling 210 such that first and second rotation housing half sections 262' and 264' may rotate about longitudinal axis "X" of adapter 200 with respect to drive coupling 210, but not translate axially along longitudinal axis "X" of adapter 200 with respect to drive coupling 210. Drive coupling 210 may be supported in annular groove 201' by any suitable means known in the art, such as by a bushing, ball bearing, or the like. In one non-limiting embodiment, annular groove 201' receives drive coupling 210 by means of a ball bearing 207'. Ball bearing 207' may be fixed to drive coupling 210 by any suitable means known in the art, such as welding, friction fit, adhesives, or the like. In one non-limiting embodiment, ball bearing 207' is fixed to drive coupling 210 by means of welding. The addition of annular groove 201' and ball bearing 207', in combination with the features already disclosed in previous embodiments, serve to axially locate drive coupling 210, such that drive coupling 210 is prevented from advancing axially along longitudinal axis "X" (FIG. 10) of adapter 200, thereby reducing the amount of over-articulation caused by the forces transmitted through adapter 200 during firing of the staples.

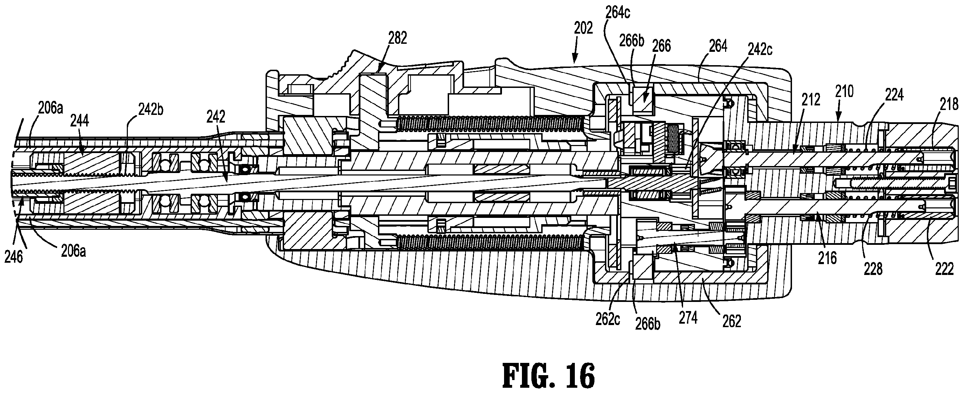

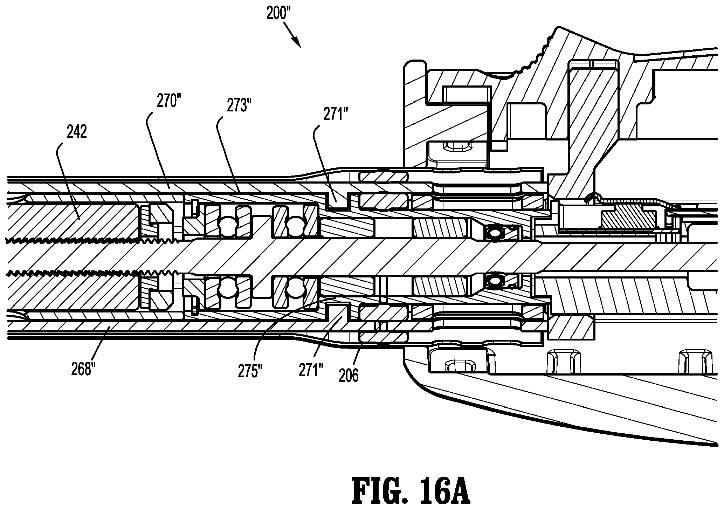

Referring to FIGS. 13A, 13B, and 16A, another embodiment of an adapter provided in accordance with the present disclosure is shown is generally designated as adapter 200". Adapter 200" is configured similar to the adapters 200 and 200' and therefore, in the interest of brevity, only the additional or alternate components thereof are described hereinafter.

Adapter 200" may include an alternate pair of rotation transmitting bars 268" and 270". Rotation transmitting bars 268" and 270" are configured similar to rotation transmitting bars 268 and 270 of above, with the exception of a proximal boss 271" (see FIG. 13A) extending from the inner surface 272" of rotation transmitting bars 268" and 270".

Adapter 200" may further include an alternate proximal end 273" of inner housing tube 206a of outer tube 206 (see FIGS. 13 and 13B). An outer surface 274" of proximal end 273" defines an annular groove 275" therein. Annular groove 275" extends radially inward towards longitudinal axis "X" of adapter 200 and is configured to receive proximal boss 271" of alternate rotation transmitting bars 268" and 270" (see FIG. 16A). The combination of proximal end 273" and rotation transmitting bars 268" and 270", when used in combination with the features already disclosed in previous embodiments, serves to axially locate first distal drive shaft 242 such that first distal drive shaft 242 is prevented from advancing axially along longitudinal axis "X" of adapter 200, thereby reducing the amount of over-articulation caused by the forces transmitted through adapter 200 during firing of end effector 300.

As illustrated in FIGS. 1 and 21, the end effector is designated as 300. End effector 300 is configured and dimensioned for endoscopic insertion through a cannula, trocar or the like. In particular, in the embodiment illustrated in FIGS. 1 and 21, end effector 300 may pass through a cannula or trocar when end effector 300 is in a closed condition.

End effector 300 includes a proximal body portion 302 and a tool assembly 304. Proximal body portion 302 is releasably attached to a distal coupling 230 of adapter 200 and tool assembly 304 is pivotally attached to a distal end of proximal body portion 302. Tool assembly 304 includes an anvil assembly 306 and a cartridge assembly 308. Cartridge assembly 308 is pivotal in relation to anvil assembly 306 and is movable between an open or unclamped position and a closed or clamped position for insertion through a cannula of a trocar.

Proximal body portion 302 includes at least a drive assembly 360 and an articulation link 366.

Referring to FIG. 21, drive assembly 360 includes a flexible drive beam 364 having a distal end which is secured to a dynamic clamping member 365, and a proximal engagement section 368. Engagement section 368 includes a stepped portion defining a shoulder 370. A proximal end of engagement section 368 includes diametrically opposed inwardly extending fingers 372. Fingers 372 engage a hollow drive member 374 to fixedly secure drive member 374 to the proximal end of beam 364. Drive member 374 defines a proximal porthole 376 which receives connection member 247 of drive tube 246 of first drive converter assembly 240 of adapter 200 when end effector 300 is attached to distal coupling 230 of adapter 200.

When drive assembly 360 is advanced distally within tool assembly 304, an upper beam of clamping member 365 moves within a channel defined between anvil plate 312 and anvil cover 310 and a lower beam moves over the exterior surface of carrier 316 to close tool assembly 304 and fire staples therefrom.

Proximal body portion 302 of end effector 300 includes an articulation link 366 having a hooked proximal end 366a which extends from a proximal end of end effector 300. Hooked proximal end 366a of articulation link 366 engages coupling hook 258c of drive bar 258 of adapter 200 when end effector 300 is secured to distal housing 232 of adapter 200. When drive bar 258 of adapter 200 is advanced or retracted as described above, articulation link 366 of end effector 300 is advanced or retracted within end effector 300 to pivot tool assembly 304 in relation to a distal end of proximal body portion 302.

As illustrated in FIG. 21, cartridge assembly 308 of tool assembly 304 includes a staple cartridge 305 supportable in carrier 316. Staple cartridge 305 defines a central longitudinal slot 305a, and three linear rows of staple retention slots 305b positioned on each side of longitudinal slot 305a. Each of staple retention slots 305b receives a single staple 307 and a portion of a staple pusher 309. During operation of surgical device 100, drive assembly 360 abuts an actuation sled and pushes actuation sled through cartridge 305. As the actuation sled moves through cartridge 305, cam wedges of the actuation sled sequentially engage staple pushers 309 to move staple pushers 309 vertically within staple retention slots 305b and sequentially eject a single staple 307 therefrom for formation against anvil plate 312.

Reference may be made to U.S. Patent No. 7,819,896, entitled "TOOL ASSEMBLY FOR A SURGICAL STAPLING DEVICE" for a detailed discussion of the construction and operation of end effector 300.

It will be understood that various modifications may be made to the embodiments of the presently disclosed adapter assemblies. Therefore, the above description should not be construed as limiting, but merely as exemplifications of embodiments. Those skilled in the art will envision other modifications within the scope of the present disclosure.

## Claims

1. An adapter (200') for selectively interconnecting a surgical end effector (300) that is configured to perform a function and a handle assembly (102) that is configured to actuate the end effector, the end effector including at least one axially translatable drive member (374), and the handle assembly including at least one rotatable drive shaft (212), the adapter comprising:
an adapter housing (202') configured and adapted for connection with the handle assembly and to be in operative communication with each rotatable drive shaft of the handle assembly;
an outer tube (206) having a proximal end (204) and a distal end (205), wherein the distal end of the outer tube is configured and adapted for connection with the end effector (300) and for operative communication with each axially translatable drive member of the end effector;
an inner housing (262', 264') having proximal and distal ends defining a longitudinal axis wherein the inner housing is rotatably supported in the adapter housing; and
at least one drive converter assembly for interconnecting a respective one rotatable drive shaft of the handle assembly and one axially translatable drive member of the end effector, wherein the at least one drive converter assembly includes a first end that is connectable to a rotatable drive shaft of the handle assembly and a second end that is connectable to the axially translatable drive member of the end effector, wherein the at least one drive converter assembly converts and transmits a rotation of the rotatable drive shaft of the handle assembly to an axial translation of the axially translatable drive member of the end effector; **characterised in that**;
the adapter housing (202') has an inner wall surface defining a cavity, wherein a flange (203') projects radially inward from the inner wall surface on the distal end of the adapter housing;
the outer tube (206) tapers distally from a larger diameter to a smaller diameter, the outer tube rotatably and translatably supported by the flange (203') of the adapter housing,
the inner housing (262', 264') having an inner wall surface (208') defining a cavity and an outer surface, wherein an annular groove (201') is defined in the proximal end of the inner wall surface (208'), wherein a diameter of the outer surface of the inner housing is less than a diameter of the inner wall surface of the adapter housing, and
wherein the at least one drive converter assembly is configured to be rotatably and translatably supported in the annular groove (201') in the inner housing.

2. The adapter according to Claim 1, wherein the inner housing (262', 264') is in mechanical cooperation with the outer tube (206); preferably wherein a plurality of arms (262a, 264a) extends distally along the longitudinal axis from the inner housing and are in mechanical cooperation with the outer tube; preferably still wherein the plurality of arms comprises a pair of parallel arms.

3. The adapter according to Claim 1 or claim 2, wherein the at least one drive converter assembly of the adapter includes a first drive converter assembly (240) including:
a first distal drive shaft (242) rotatably supported in the adapter housing, wherein a proximal end of the first distal drive shaft is connectable to the rotatable drive shaft of the handle assembly;
a drive coupling nut (244) threadably connected to a threaded distal portion of the first distal drive shaft, wherein the drive coupling nut is keyed against rotation within the adapter housing; and
a drive tube (246) having a proximal end connected to the drive coupling nut (244) and a distal end configured for selective engagement with the at least one axially translatable drive member (374) of the end effector;
wherein rotation of the rotatable drive shaft (212) of the handle assembly results in rotation of the distal drive shaft, and wherein rotation of the distal drive shaft (242) results in axial translation of the drive coupling nut (244), the drive tube (246) and the at least one axially translatable drive member (374) of the end effector,

4. The adapter according to claim 3, wherein the first drive converter assembly includes:
a spur gear (242c) keyed to the proximal end of the distal drive shaft (242);
a proximal rotatable drive shaft (212) having a spur gear (212a) supported on a distal end thereof and a proximal end connectable to the rotatable drive shaft of the handle assembly; and
a compound gear (243) interengaging the spur gear (242c) keyed to the proximal end of the distal drive shaft and the spur gear (242a) supported on the distal end of the proximal rotatable drive shaft.

5. The adapter according to claim 4, wherein translation of the at least one axially translatable drive member of the end effector results in a closing of the end effector and a firing of the end effector.

6. The adapter according to claim 4 or claim 5, wherein the at least one drive converter assembly of the adapter includes a second drive converter assembly (250) including:
a second proximal drive shaft (214) rotatably supported in the adapter housing, wherein a proximal end (214a) of the second proximal drive shaft is connectable to a second rotatable drive shaft of the handle assembly.
a coupling cuff (254) rotatably and translatably supported in the adapter housing, the coupling cuff defining an inner annular race;
a coupling slider (256) rotatably disposed within the annular race of the coupling cuff, the coupling slider being threadably connected to a threaded distal portion of the second proximal drive shaft; and
a drive bar (258) having a proximal end connected to the coupling cuff and a distal end configured for selective engagement with another raxially translatably drive member of the end effector;
wherein rotation of the second rotatably drive shaft of the handle assembly results in rotation of the second proximal drive shaft, and wherein rotation of the second proximal drive shaft results in axial translation of the coupling slider, the coupling cuff, the drive bar and the another axially translatable drive member of the end effector.

7. The adapter according to claim 6, wherein the first distal drive shaft extends through the coupling cuff (254) such that the coupling cuff is rotatable about the first distal drive shaft; and/or wherein translation of the another axially translatable drive member of the end effector results in an articulation of the end effector relative to the adapter.

8. The adapter according to any preceding claim, wherein the adapter further comprises a drive transmitting assembly (260) including:
a third proximal rotatable drive shaft (216) rotatably supported in the adapter housing and having a spur gear (216a) supported on a distal end thereof and a proximal end connectable to a third rotatable drive shaft of the handle assembly;
a ring gear (266) rotatably supported in the adapter housing, the ring gear defining an internal array of gear teeth (266a) which are engaged with the spur gear of the third proximal rotatable drive shaft, wherein the ring gear is keyed to the inner housing;
at least one rotation transmitting bar (268, 270) having an proximal end connected to the inner housing and a distal end connected to a distal coupling assembly, wherein the distal coupling assembly is configured to selectively connect with the end effector;
wherein rotation of the third rotatable drive shaft of the handle assembly results in rotation of the third proximal drive shaft, and wherein rotation of the third proximal drive shaft results in rotation of the ring gear, the inner housing, the at least one rotation transmitting bar and the distal coupling assembly to rotate the end effector relative to the adapter and about a longitudinal axis defined by the adapter.

9. The adapter according to claim 8, wherein the outer tube (206) of the adapter is configured for endoscopic insertion into a target surgical site.

10. The adapter according to any preceding claim, wherein the adapter housing (202) is inhibited from insertion into the target surgical site.

11. The adapter according to claim 10, wherein at least one of the first drive converter assembly, the second drive converter assembly and the drive transmitting assembly is disposed in the adapter housing.

12. The adapter according to claim 11, wherein the end effector (300) and the outer tube (206) of the adapter define an endoscopic portion that is configured for endoscopic insertion into a target surgical site.

13. The adapter according to claim 12, wherein the end effector (300) and the outer tube (206) define an endoscopic portion that is configured for endoscopic insertion into a target surgical site.

14. The adapter according to claim 13, wherein each of the first drive converter assembly, and the second drive converter assembly and the drive transmitting assembly is disposed outside of the endoscopic portion.

## Patentansprüche

1. Adapter (200') zum wahlweise Verbinden eines chirurgischen Endeffektors (300), der konfiguriert ist, um eine Funktion zu erfüllen, und einer Handgriffbaugruppe (102), die konfiguriert ist, um den Endeffektor zu betätigen, wobei der Endeffektor zumindest ein translatierbares Antriebsglied (374) aufweist und die Handgriffbaugruppe zumindest einen drehbaren Antriebsschaft (212) aufweist, wobei der Adapter umfasst:
ein Adaptergehäuse (202'), das für eine Verbindung mit der Handgriffbaugruppe und um in funktionsfähiger Verbindung mit jedem drehbaren Antriebsschaft der Handgriffbaugruppe zu sein konfiguriert und angepasst ist;
ein äußeres Rohr (206) mit einem proximalen Ende (204) und einem distalen Ende (205), wobei das distale Ende des äußeren Rohres für eine Verbindung mit dem Endeffektor (300) und für eine funktionsfähige Verbindung mit jedem translatierbaren Antriebsglied des Endeffektors konfiguriert und angepasst ist;
ein inneres Gehäuse (262', 264') mit proximalen und distalen Enden, die eine Längsachse definieren, wobei das innere Gehäuse in dem Adaptergehäuse drehbar gestützt ist; und
zumindest eine Antriebsumwandleranordnung zum Verbinden eines entsprechenden drehbaren Antriebsschafts der Handgriffbaugruppe und eines translatierbaren Antriebsgliedes des Endeffektors, wobei die zumindest eine Antriebsumwandleranordnung ein erstes Ende, das mit einem drehbaren Antriebsschaft der Handgriffbaugruppe verbunden werden kann, und ein zweites Ende, das mit dem translatierbaren Antriebsglied des Endeffektors verbunden werden kann, aufweist, wobei die zumindest eine Antriebsumwandleranordnung eine Drehung des drehbaren Antriebsschafts der Handgriffbaugruppe in eine Translation des translatierbaren Antriebsglieds des Endeffektors umwandelt und überträgt, **dadurch gekennzeichnet, dass**
das Adaptergehäuse (202') eine innere Wandfläche hat, die einen Hohlraum definiert, wobei ein Flansch (203') von der inneren Wandfläche an dem distalen Ende des Adaptergehäuses radial nach innen vorsteht;
sich das äußere Rohr (206) von einem größeren Durchmesser zu einem kleineren Durchmesser distal verjüngt, wobei das äußere Rohr von dem Flansch (203') des Adaptergehäuses drehbar und translatierbar gestützt ist,
das innere Gehäuse (262', 264') eine innere Wandfläche (208'), die einen Hohlraum definiert, und eine äußere Fläche hat, wobei eine ringförmige Rille (201') in dem proximalen Ende der inneren Wandfläche (208') definiert ist, wobei ein Durchmesser der äußeren Fläche des inneren Gehäuses geringer als ein Durchmesser der inneren Wandfläche des Adaptergehäuses ist; und
wobei die zumindest eine Antriebsumwandleranordnung konfiguriert ist, um in der ringförmigen Rille (201') in dem inneren Gehäuse drehbar und translatierbar gestützt zu werden.

2. Adapter nach Anspruch 1, wobei das innere Gehäuse (262', 264') mit dem äußeren Rohr (206) mechanisch zusammenwirkt, wobei sich bevorzugt eine Vielzahl von Armen (262a, 264a) distal entlang der Längsachse von dem inneren Gehäuse erstrecken und mit dem äußeren Rohr mechanisch zusammenwirken, wobei die Vielzahl von Armen noch bevorzugter ein Paar von parallelen Armen umfasst.

3. Adapter nach Anspruch 1 oder Anspruch 2, wobei die zumindest eine Antriebsumwandleranordnung des Adapters eine erste Antriebsumwandleranordnung (240) aufweist, aufweisend:
einen ersten distalen Antriebsschaft (242), der in dem Adaptergehäuse drehbar gestützt ist, wobei ein proximales Ende des ersten distalen Antriebsschafts mit dem drehbaren Antriebsschaft der Handgriffbaugruppe verbunden werden kann;
eine Antriebskupplungsmutter (244), die mit einem distalen Gewindeabschnitt des ersten distalen Antriebsschafts schraubbar verbunden ist, wobei die Antriebskupplungsmutter innerhalb des Adaptergehäuses verdrehsicher verkeilt ist; und
ein Antriebsrohr (246) mit einem proximalen Ende, das mit der Antriebskupplungsmutter (244) verbunden ist, und einem distalen Ende, das für einen wahlweise Eingriff mit dem zumindest einen translatierbaren Antriebsglied (374) des Endeffektors konfiguriert ist;
wobei eine Drehung des drehbaren Antriebsschafts (212) der Handgriffbaugruppe eine Drehung des distalen Antriebsschafts zur Folge hat und wobei eine Drehung des distalen Antriebsschafts (242) eine axiale Translation der Antriebskupplungsmutter (244), des Antriebsrohres (246) und des zumindest einen translatierbaren Antriebsglieds (374) des Endeffektors zur Folge hat.

4. Adapter nach Anspruch 3, wobei die erste Antriebsumwandleranordnung aufweist:
ein Stirnrad (242c), das mit dem proximalen Ende des distalen Antriebsschafts (242) verkeilt ist;
einen proximalen drehbaren Antriebsschaft (212) mit einem Stirnrad (212a), das an einem distalen Ende davon gestützt ist, und einem proximalen Ende, das mit dem drehbaren Antriebsschaft der Handgriffbaugruppe verbunden werden kann; und
ein Verbundrad (243), das mit dem Stirnrad (242c), das mit dem proximalen Ende des distalen Antriebsschafts verkeilt ist, und dem Stirnrad (242a), das an dem distalen Ende des proximalen drehbaren Antriebsschafts gestützt ist, ineinand ergreift.

5. Adapter nach Anspruch 4, wobei eine Translation des zumindest einen translatierbaren Antriebsglieds des Endeffektors ein Schließen des Endeffektors und ein Auslösen des Endeffektors zur Folge hat.

6. Adapter nach Anspruch 4 oder Anspruch 5, wobei die zumindest eine Antriebsumwandleranordnung des Adapters eine zweite Antriebsumwandleranordnung (250) aufweist, aufweisend:
einen zweiten proximalen Antriebsschaft (214), der in der Adapteranordnung drehbar gestützt ist, wobei ein proximales Ende (214a) des zweiten proximalen Antriebsschafts mit einem zweiten drehbaren Antriebsschaft der Handgriffbaugruppe verbunden werden kann;
eine Kupplungsmanschette (254), die in dem Adaptergehäuse drehbar und translatierbar gestützt ist, wobei die Kupplungsmanschette einen inneren ringförmigen Laufring definiert;
einen Kupplungsschieber (256), der innerhalb des ringförmigen Laufrings der Kupplungsmanschette drehbar angeordnet ist, wobei der Kupplungsschieber mit einem distalen Gewindeabschnitt des zweiten proximalen Antriebsschafts schraubbar verbunden ist; und
einen Antriebsbalken (258) mit einem proximalen Ende, das mit der Kupplungsmanschette verbunden ist, und einem distalen Ende, das für einen wahlweise Eingriff mit einem anderen translatierbaren Antriebsglied des Endeffektors konfiguriert ist;
wobei eine Drehung des zweiten drehbaren Antriebsschafts der Handgriffbaugruppe eine Drehung des zweiten proximalen Antriebsschafts zur Folge hat und wobei eine Drehung des zweiten proximalen Antriebsschafts eine Translation des Kupplungsschiebers, der Kupplungsmanschette, des Antriebsbalkens und des anderen translatierbaren Antriebsglieds des Endeffektors zur Folge hat.

7. Adapter nach Anspruch 6, wobei sich der erste distale Antriebsschaft durch die Kupplungsmanschette (254) erstreckt, so dass die Kupplungsmanschette um den ersten distalen Antriebsschaft drehbar ist, und/oder wobei eine Translation des anderen translatierbaren Antriebsglieds des Endeffektors eine Gelenkbetätigung des Endeffektors relativ zu dem Adapter zur Folge hat.

8. Adapter nach einem vorstehenden Anspruch, wobei der Adapter weiter eine Antriebsübertragungsanordnung (260) umfasst, aufweisend:
einen dritten proximalen drehbaren Antriebsschaft (216), der in dem Adaptergehäuse drehbar gestützt ist und mit einem Stirnrad (216a), das an einem distalen Ende davon gestützt ist, und einem proximalen Ende, das mit einem dritten drehbaren Antriebsschaft der Handgriffbaugruppe verbunden werden kann;
ein Ringrad (266), das in dem Adaptergehäuse drehbar gestützt ist, wobei das Ringrad eine interne Anordnung von Radzähnen (266a) definiert, die mit dem Stirnrad des dritten proximalen drehbaren Antriebsschafts in Eingriff stehen, wobei das Ringrad an dem inneren Gehäuse verkeilt ist;
zumindest einen Drehübertragungsbalken (268, 270) mit einem proximalen Ende, das mit dem inneren Gehäuse verbunden ist, und einem distalen Ende, das mit einer distalen Kupplungsanordnung verbunden ist, wobei die distale Kupplungsanordnung konfiguriert ist, um sich wahlweise mit dem Endeffektor zu verbinden;
wobei eine Drehung des dritten drehbaren Antriebsschafts der Handgriffbaugruppe eine Drehung des dritten proximalen Antriebsschafts zur Folge hat und wobei eine Drehung des dritten proximalen Antriebsschafts eine Drehung des Ringrads, des inneren Gehäuses, des zumindest einen Drehübertragungsbalkens und der distalen Kupplungsanordnung zur Folge hat, um den Endeffektor relativ zu dem Adapter und um eine durch den Adapter definierte Längsachse zu drehen.

9. Adapter nach Anspruch 8, wobei das äußere Rohr (206) des Adapters für eine endoskopische Einbringung in einen chirurgischen Zielort konfiguriert ist.

10. Adapter nach einem vorstehenden Anspruch, wobei das Adaptergehäuse (202) an einer Einbringung in den chirurgischen Zielort gehindert wird.

11. Adapter nach Anspruch 10, wobei zumindest eine von der ersten Antriebsumwandleranordnung, der zweiten Antriebsumwandleranordnung und der Antriebsübertragungsanordnung in dem Adaptergehäuse angeordnet ist.

12. Adapter nach Anspruch 11, wobei der Endeffektor (300) und das äußere Rohr (206) des Adapters einen endoskopischen Abschnitt definieren, der für eine endoskopische Einbringung in einen chirurgischen Zielort konfiguriert ist.

13. Adapter nach Anspruch 12, wobei der Endeffektor (300) und das äußere Rohr (206) einen endoskopischen Abschnitt definieren, der für eine endoskopische Einbringung in einen chirurgischen Zielort konfiguriert ist.

14. Adapter nach Anspruch 13, wobei jede von der ersten Antriebsumwandleranordnung und der zweiten Antriebsumwandleranordnung und der Antriebsübertragungsanordnung außerhalb des endoskopischen Abschnitts angeordnet ist.

## Revendications

1. Adaptateur (200') pour interconnecter de manière sélective un effecteur d'extrémité chirurgical (300) qui est configuré pour effectuer une fonction et un ensemble poignée (102) qui est configuré pour actionner l'effecteur d'extrémité, l'effecteur d'extrémité incluant au moins un élément d'entraînement (374) pouvant subir une translation axiale et l'ensemble poignée incluant au moins un arbre d'entraînement rotatif (212), l'adaptateur comprenant :
un logement d'adaptateur (202') configuré et conçu pour connexion à l'ensemble poignée et pour être en communication opérationnelle avec chaque arbre d'entraînement rotatif de l'ensemble poignée ;
un tube extérieur (206) ayant une extrémité proximale (204) et une extrémité distale (205), dans lesquelles l'extrémité distale du tube extérieur est configurée et conçue pour connexion à l'effecteur d'extrémité (300) et pour communication opérationnelle avec chaque élément d'entraînement pouvant subir une translation axiale de l'effecteur d'extrémité ;
un logement intérieur (262', 264') ayant des extrémités proximale et distale définissant un axe longitudinal dans lequel le logement intérieur est supporté en rotation dans le logement d'adaptateur ; et
au moins un ensemble convertisseur d'entraînement pour interconnecter un arbre d'entraînement respectif rotatif de l'ensemble poignée et un élément d'entraînement pouvant subir une translation axiale de l'effecteur d'extrémité, dans lequel l'au moins un ensemble convertisseur d'entraînement inclut une première extrémité qui peut être reliée à un arbre d'entraînement rotatif de l'ensemble poignée et une seconde extrémité qui peut être reliée à l'élément d'entraînement pouvant subir une translation axiale de l'effecteur d'extrémité, dans lequel l'au moins un ensemble convertisseur d'entraînement transforme et transmet une rotation de l'arbre d'entraînement rotatif de l'ensemble poignée à une translation axiale de l'élément d'entraînement pouvant subir une translation axiale de l'effecteur d'extrémité; **caractérisé en ce que** ;
le logement d'adaptateur (202') a une surface de paroi intérieure définissant une cavité, dans lequel une bride (203') dépasse de façon radiale vers l'intérieur à partir de la surface de paroi intérieure sur l'extrémité distale du logement d'adaptateur ;
le tube extérieur (206) va en s'amincissant de façon distale à partir d'un diamètre plus grand jusqu'à un diamètre plus petit, le tube extérieur étant supporté en rotation et de manière à pouvoir subir une translation par la bride (203') du logement d'adaptateur,
le logement intérieur (262', 264') ayant une surface de paroi intérieure (208') définissant une cavité et une surface extérieure, dans lequel une rainure annulaire (201') est définie dans l'extrémité proximale de la surface de paroi intérieure (208'), dans lequel un diamètre de la surface extérieure du logement intérieur est inférieur à un diamètre de la surface de paroi intérieure du logement d'adaptateur, et
dans lequel l'au moins un ensemble convertisseur d'entraînement est configuré pour être supporté en rotation et de manière à pouvoir subir une translation dans la rainure annulaire (201') dans le logement intérieur.

2. Adaptateur selon la revendication 1, dans lequel le logement intérieur (262', 264') est en coopération mécanique avec le tube extérieur (206) ; de préférence dans lequel une pluralité de bras (262a, 264a) s'étendent de façon distale le long de l'axe longitudinal à partir du logement intérieur et sont en coopération mécanique avec le tube extérieur; de préférence toujours dans lequel la pluralité de bras comprend un couple de bras parallèles.

3. Adaptateur selon la revendication 1 ou la revendication 2, dans lequel l'au moins un ensemble convertisseur d'entraînement de l'adaptateur inclut un premier ensemble convertisseur d'entraînement (240) incluant :
un premier arbre d'entraînement distal (242) supporté en rotation dans le logement d'adaptateur, dans lequel une extrémité proximale du premier arbre d'entraînement distal peut être reliée à l'arbre d'entraînement rotatif de l'ensemble poignée ;
un écrou d'accouplement d'entraînement (244) relié par filetage à une portion distale filetée du premier arbre d'entraînement distal, dans lequel l'écrou d'accouplement d'entraînement est verrouillé contre la rotation à l'intérieur du logement d'adaptateur ; et
un tube d'entraînement (246) ayant une extrémité proximale reliée à l'écrou d'accouplement d'entraînement (244) et une extrémité distale configurée pour mise en prise sélective avec l'au moins un élément d'entraînement (374) pouvant subir une translation axiale de l'effecteur d'extrémité ;
dans lequel la rotation de l'arbre d'entraînement rotatif (212) de l'ensemble poignée résulte en la rotation de l'arbre d'entraînement distal, et dans lequel la rotation de l'arbre d'entraînement distal (242) résulte en une translation axiale de l'écrou d'accouplement d'entraînement (244), le tube d'entraînement (246) et l'au moins un élément d'entraînement (374) pouvant subir une translation axiale de l'effecteur d'extrémité.

4. Adaptateur selon la revendication 3, dans lequel le premier ensemble convertisseur d'entraînement inclut :
un engrenage cylindrique (242c) verrouillé sur l'extrémité proximale de l'arbre d'entraînement distal (242) ;
un arbre d'entraînement rotatif proximal (212) ayant un engrenage cylindrique (212a) supporté sur une extrémité distale de ce dernier et une extrémité proximale pouvant être reliée à l'arbre d'entraînement rotatif de l'ensemble poignée ; et
un engrenage composé (243) mettant mutuellement en prise l'engrenage cylindrique (242c) verrouillé sur l'extrémité proximale de l'arbre d'entraînement distal et l'engrenage cylindrique (242a) supporté sur l'extrémité distale de l'arbre d'entraînement rotatif proximal.

5. Adaptateur selon la revendication 4, dans lequel la translation de l'au moins un élément d'entraînement pouvant subir une translation axiale de l'effecteur d'extrémité résulte en une fermeture de l'effecteur d'extrémité et en un tir de l'effecteur d'extrémité.

6. Adaptateur selon la revendication 4 ou la revendication 5, dans lequel l'au moins un ensemble convertisseur d'entraînement de l'adaptateur inclut un second ensemble convertisseur d'entraînement (250) incluant :
un deuxième arbre d'entraînement proximal (214) supporté en rotation dans le logement d'adaptateur, dans lequel une extrémité proximale (214a) du deuxième arbre d'entraînement proximal peut être reliée à un deuxième arbre d'entraînement rotatif de l'ensemble poignée ;
une manchette d'accouplement (254) supportée en rotation et de manière à pouvoir subir une translation dans le logement d'adaptateur, la manchette d'accouplement définissant un chemin de roulement annulaire intérieur ;
un coulisseau d'accouplement (256) disposé en rotation à l'intérieur du chemin de roulement annulaire de la manchette d'accouplement, le coulisseau d'accouplement étant relié par filetage à une portion distale filetée du deuxième arbre d'entraînement proximal ; et
une barre d'entraînement (258) ayant une extrémité proximale reliée à la manchette d'accouplement et une extrémité distale configurée pour engagement sélectif avec un autre élément d'entraînement pouvant subir une translation axiale de l'effecteur d'extrémité ;
dans lequel la rotation du deuxième arbre d'entraînement rotatif de l'ensemble poignée résulte en la rotation du deuxième arbre d'entraînement proximal, et dans lequel la rotation du deuxième arbre d'entraînement proximal résulte en la translation axiale du coulisseau d'accouplement, de la manchette d'accouplement, de la barre d'entraînement et d'un autre élément d'entraînement pouvant subir une translation axiale de l'effecteur d'extrémité.

7. Adaptateur selon la revendication 6, dans lequel le premier arbre d'entraînement distal s'étend à travers la manchette d'accouplement (254) de sorte que la manchette d'accouplement est rotative autour du premier arbre d'entraînement distal; et/ou dans lequel la translation du un autre élément d'entraînement pouvant subir une translation axiale de l'effecteur d'extrémité résulte en une articulation de l'effecteur d'extrémité par rapport à l'adaptateur.

8. Adaptateur selon l'une quelconque des revendications précédentes, dans lequel l'adaptateur comprend en outre un ensemble de transmission d'entraînement (260) incluant :
un troisième arbre d'entraînement rotatif proximal (216) supporté en rotation dans le logement d'adaptateur et ayant un engrenage cylindrique (216a) supporté sur une extrémité distale de ce dernier et une extrémité proximale pouvant être reliée à un troisième arbre d'entraînement rotatif de l'ensemble poignée ;
une couronne de train planétaire (266) supportée en rotation dans le logement d'adaptateur, la couronne de train planétaire définissant un groupement interne de dents d'engrenage (266a) qui sont en prise avec l'engrenage cylindrique du troisième arbre d'entraînement rotatif proximal, dans lequel la couronne de train planétaire est verrouillée sur le logement intérieur ;
au moins une barre de transmission de rotation (268, 270) ayant une extrémité proximale reliée au logement intérieur et une extrémité distale reliée à un ensemble d'accouplement distal, dans lequel l'ensemble d'accouplement distal est configuré pour être relié de manière sélective à l'effecteur d'extrémité ;
dans lequel la rotation du troisième arbre d'entraînement rotatif de l'ensemble poignée résulte en la rotation du troisième arbre d'entraînement proximal, et dans lequel la rotation du troisième arbre d'entraînement proximal résulte en la rotation de la couronne de train planétaire, du logement intérieur, l'au moins une barre de transmission de rotation et l'ensemble d'accouplement distal pour faire tourner l'effecteur d'extrémité par rapport à l'adaptateur et autour d'un axe longitudinal défini par l'adaptateur.

9. Adaptateur selon la revendication 8, dans lequel le tube extérieur (206) de l'adaptateur est configuré pour insertion endoscopique dans un site chirurgical cible.

10. Adaptateur selon l'une quelconque des revendications précédentes, dans lequel l'insertion du logement d'adaptateur (202) est inhibée dans le site chirurgical cible.

11. Adaptateur selon la revendication 10, dans lequel au moins un du premier ensemble convertisseur d'entraînement, le second ensemble convertisseur d'entraînement et l'ensemble de transmission d'entraînement est disposé dans le logement d'adaptateur.

12. Adaptateur selon la revendication 11, dans lequel l'effecteur d'extrémité (300) et le tube extérieur (206) de l'adaptateur définissent une portion endoscopique qui est configurée pour insertion endoscopique dans un site chirurgical cible.

13. Adaptateur selon la revendication 12, dans lequel l'effecteur d'extrémité (300) et le tube extérieur (206) définissent une portion endoscopique qui est configurée pour insertion endoscopique dans un site chirurgical cible.

14. Adaptateur selon la revendication 13, dans lequel chacun du premier ensemble convertisseur d'entraînement et du second ensemble convertisseur d'entraînement et de l'ensemble de transmission d'entraînement est disposé à l'extérieur de la portion endoscopique.
